# EUROPEAN PATENT SPECIFICATION

(11) **EP 1 492 475 B1**
(45) Date of publication and mention of the grant of the patent: **21.12.2011**
(21) Application number: 02725688.2
(22) Date of filing: 16.04.2002
(51) Int. Cl.: A61L 27/42, A61L 27/56

(54) **DENSE/POROUS STRUCTURES FOR USE AS BONE SUBSTITUTES**
DICHTE/PORÖSE STRUKTUREN ZUR VERWENDUNG ALS KNOCHENERSATZ
STRUCTURES DENSES/POREUSES UTILISEES COMME SUBSTITUTS OSSEUX

(30) Priority: 16.04.2001 US 283752 P
(43) Date of publication of application: 05.01.2005
(73) Proprietor: Wright Medical Technology, Inc., Arlington, Tennessee 38002 (US)
(72) Inventor: CASSIDY, James, J., Shakopee, MN 55379 (US); NORBERG, Brian, L., Durand, WI 54736 (US); HECKENDORF, Bradley, R., Menomonie, WI 54751 (US); KO, Ying, Hudson, WI 54016 (US)
(74) Representative: Zounek, Nikolai
(86) International application number: PCT/US2002/011909
(87) International publication number: WO 2002/083188

(56) References cited:
- EP-A- 0 328 041
- WO-A-99/16478
- US-A- 5 152 791
- US-A- 5 783 248
- US-B1- 6 296 667

## Description

### BACKGROUND OF THE INVENTION

### 1. Field of The Invention

The present invention relates in general to dense/porous structures, in particular those made of ceramics and a process for producing the same. The present invention also relates bone substitute materials and delivery devices, preferably sustained release, for physiologically active agents and processes for producing them.

### 2. Description of Related Art

The combination of elements with one having a greater density than the other is known. See, e.g., U.S. Patent Nos. 4,447,558 to Huebsch, III, 4,158,684 to Klawitter et al., and 5,015,610 to Dwivedi. Some are disclosed being made of ceramics. See, e.g., the '684 patent to Klawitter et al. and U.S. Patent No. 5,192,325 to Kijima et al. Some of these are disclosed as being useful in bone repair. See, e.g. the '684 patent and U.S. Patent No. 6,149, 688. However, this prior art fails to "mimic" natural bone in that it does not have the proper geometry, porosity, openness, or fails to satisfactorily join the dense and porous elements, and thus has disadvantages for use as bone substitute materials. U.S. Patent No. 6,136,029 and patent WO 99/16478 , assigned to the present assignee, discloses material useful as bone substitutes. WO 01/12106 describes shaped bodies that include a porous portion produced by a redox precipitation reaction (RPR). WO '106 also discloses composite bodies that include the porous portion and a solid portion that can be formed from a wide range of materials such as metals, ceramic, glass, polymers or other hard materials. The use of acrylic polymers as the hard materials is exemplified.

US 5 783 248 discloses a process to produce a bioceramic composite made of a porous calcium phosphate material that is cemented to a dense alumina based ceramic material. The natural bone material is heated at about 700°C, then the bone is coated with a calcium phosphate material and placed on a layer of calcium phosphate. The calcium phosphate layer is on top of the alumina based ceramic substrate and the composite is then sintered to form the bioceramic composite.

In addition to bone substitute materials described above, there are other applications in which the chemical, thermal, or other properties of a ceramic, metal, or other material can best be used in a combination dense/porous form. One example is in the field of sustained release drug delivery.

### SUMMARY OF THE INVENTION

One object of the invention is to overcome the disadvantages of the known art described above. Another object of the invention is to provide a dense/porous structure that better mimics the characteristics of natural bone. Still another object of the invention is to provide a method for producing a dense/porous structure. Another object of the invention is to provide a dense/porous structure that has improved joining between the dense and porous elements. Yet another object of the invention is to provide an improved delivery system, preferably sustained release, for physiologically active agents. In order to achieve the foregoing and further objects, there has been provided according to one aspect of the invention, a combination dense/porous structure, that includes: a porous element having an outer surface defining a shape having a bulk volume, the element comprising a continuous framework having struts defining a plurality of interconnecting interstices throughout the bulk volume, and said porous element having interconnecting interstices extending throughout the volume and opening through the surface; a dense element formed from a material and having a sintered density of at least 95% contacting at least a portion of the porous element; and an interconnection zone formed by the inter-penetration of the material forming the dense element into the porous element.

According to another aspect of the invention, there has been provided a combination dense/porous structure useful as a bone substitute material comprising a combination dense/porous structure described above.

According to still another aspect of the invention, there has been provided combination dense/porous structures useful as an artificial bone structure comprising an element having a shape of natural bone or a portion of natural bone and a cross-section that includes the combination dense/porous structure described above that includes an inner porous portion formed from the porous element to mimic the cancellous structure of bone; and an outer dense portion completely surrounding the inner porous portion formed from the dense element to mimic the cortical structure of bone.

Another aspect of the invention provides a sustained release delivery system that includes: a reticulated, porous element having an open, interconnected porosity; and a dense element surrounding at least a portion of the porous element, wherein at least one of the dense or porous elements contains a physiologically active agent.

According to another aspect of the invention, there has been provided a combination dense/porous structure that includes: a porous element, optionally sintered, having an outer surface defining a shape having a bulk volume, the element comprising a continuous framework having struts defining a plurality of interconnecting interstices throughout the bulk volume, and said porous element having interconnecting interstices extending throughout said volume and opening through said surface; a dense element, optionally sintered, formed from a material and having a sintered density of at least 95 % , contacting at least a portion of the porous element; and an interconnection zone joining at least one surface of the porous element and dense element whereby the bonding phase penetrates into the porous element. This combination dense/porous structure can be made by a process that includes

(a) providing a porous element, optionally sintered, having an outer surface defining a shape having a bulk volume, the element comprising a framework having struts defining a plurality of interconnecting interstices throughout the bulk volume, and the article having interconnecting interstices extending throughout the volume and opening through the surface;

(b) providing a dense element, optionally sintered, formed from a material and having a sintered density of at least 95% ;

(c) providing a bonding phase;

(d) joining at least one surface of the porous element and the dense element with the bonding phase. The bonding phase penetrates into the porous element and forms an inter-penetration zone;

(e) curing and/or drying and/or heating the bonding phase to form the combination dense/porous structure; and

(f) subjecting the joined porous element and dense element to sintering temperatures, if necessary.

Further objects, features and advantages of the present invention, will become readily apparent from detailed consideration of the preferred embodiments which follow.

### BRIEF DESCRIPTION OF THE DRAWINGS

Figure 1 is a photograph showing combination dense/porous structures in the shape of discs (green) made according to the injection molding embodiment of the invention.

Figure 2 is a photograph showing a combination dense/porous structures, where the porous elements have been compressed during injection molding and have been restored to its original shape after debinding.

Figure 3 is a photograph of a combination dense/porous structure in the shape of discs made according to the injection molding embodiment of the invention, where the discs having had channels made in the porous element are shown.

Figure 4 is a photograph showing combination dense/porous structures where the porous element has been compressed during injection molding, restored to its original state during debind and then sintered.

Figure 5 is a photograph showing a combination dense/porous structure in a green state having been molded and then removed from the injection molding machine.

Figure 6 is a photograph showing a combination dense/porous structure made according to the slip casting embodiment of the invention, where a structure that has had a dense ceramic layer sintered-bonded to the porous element is shown.

Figure 8a is a view of a femur and an artificial bone substitute to replace a segment of the diaphysis of the femur. Figure 8b is a view of a tibia and an artificial bone substitute to correct an angular deformity of the metaphysis of the tibia.

Figure 9 is a view of the bones of the feet and hands that may be substituted with an artificial bone substitute according to the present invention.

### Figure 10f shows an

artificial bone structures-used to fuse adjacent bone segments.

Figure 11 is a view of adjacent vertebrae of a spinal column.

Figures 12c and 12e are views of artificial bone structures used to fuse vertebrae.

Figure 13 is a view of an artificial bone structure where the dense element surrounds the porous element and contains holes that expose the porous element to the outer surface of the dense element.

Figures 14a and 14b are views of artificial bone structures in which the dense element(s) partially or completely surround the porous element and the dense element(s) are used to stabilize the porous element.

Figure 15 is a graph showing the alkaline phosphatase activity (mean ± standard deviation), an indicator of new bone formation, in a rat model of osteoinduction using recombinant human bone morphogenetic protein 4 (rhBMP-4) delivered from porous implants of hydroxyapatite and tricalcium phosphate.

### DETAILED DESCRIPTION OF PREFERRED EMBODIMENTS

One aspect of the invention is to provide a dense/porous structure that is particularly useful in medical applications such as bone substitutes and delivery devices, preferably sustained release, for physiologically active agents. As noted above, the prior art commonly suffers from the disadvantages in a failure to reproduce the feature (e.g., geometry, porosity, openness, strength, etc.) of natural bone. As a result, the bone substitutes of the prior art cannot provide for optimum bone in-growth, etc. Accordingly, one objective of combining dense and porous ceramic elements in a medical device is to maximize the strength of an implant using the dense component while maximizing the potential for host tissue in-growth, particularly bone, into the porous element.

The porous element of the combination dense/porous structure in the present invention can be any suitable porous element. Preferably, the porous element has an outer surface defining a shape having a bulk volume having a continuous framework having struts defining a plurality of interconnecting interstices throughout the bulk volume, the porous element having interconnecting interstices extending throughout said volume and opening through said surface. In another preferred embodiment, the porous element is a continuous strong supportive, load-bearing framework, preferably sintered.

Preferably, the porous element has a hard, strong, open framework having interstices in the size range of about 50 µm to about 1000 µm, preferably from about 200 µm to about 600 µm, and having interstitial volumes of at least about 50 % , more preferably about 70% , and most preferably at least about 80%. The material of the porous element may comprise any suitable material. For medical applications, the material is preferably a strong, hard, biologically-compatible material. These materials can include bioactive ceramic materials (e.g., hydroxyapatite, tricalcium phosphate, and fluoroapatite), ceramics (e.g., alumina and zirconia), metals and combinations of these materials. The physical combination of the two elements may also permit the combination of two or more types of ceramics, e.g., a dense bioinert ceramic like alumina with a porous bioactive ceramic such as hydroxyapatite, or hydroxyapatite dense and tricalcium phosphate porous, or hydroxyapatite dense and alumina porous. In some applications, it may be desirable to use bioactive materials described above as the material for both elements.

Metals that can be used to form the porous element include titanium, stainless steels, cobalt/chromium alloys, tantalum, titanium-nickel alloys such as Nitinol and other superelastic metal alloys. Reference is made to Itin, et al., "Mechanical Properties and Shape Memory of Porous Nitinol," Materials Characterization [32] pp. 179-187 (1994); Bobyn, et al., "Bone Ingrowth Kinetics and Interface Mechanics of a Porous Tantalum Implant Material," Transactions of the 43rd Annual Meeting, Orthopaedic Research Society, p. 758, February 9-13, 1997 San Francisco, CA; and to Pederson, et al., "Finite Element Characterization of a Porous Tantalum Material for Treatment of Avascular Necrosis," Transactions of the 43rd Annual Meeting, Orthopaedic Research Society, p. 598 February 9-13, 1997. San Francisco, CA .

In a preferred embodiment, the framework structure is formed such that the interstices themselves, on average, are wider than are the thicknesses of the struts which separate neighboring interstices. The framework is essentially completely continuous and self interconnected in three dimensions, and the interstitial portion is also essentially completely continuous and self interconnected in three dimensions. These two three dimensionally interconnected parts are intercolated with one another. This can be referred to as a 3-3 connectivity structure where the first number refers to the number of dimensions in which the framework is connected, and the second number refers to the number of dimensions in which the interstitial portion is connected. The concept of connectivity is explained at greater length in Newnham et al. "Connectivity and Piezoelectric-Pyroelectric Composites," Materials Research Bulletin, Vol. 13 pp. 525-536 (1978) . With the framework described according to this embodiment, the framework itself is given a 3 as it is connected in 3 dimensions, and the interstitial portion is treated likewise. The resulting structure is a reticulum or reticulated structure. In contrast, partially sintered assemblages of powders invariably contain isolated pores which are not connected to all other pores. A material with all isolated (that is, dead end) pores in a dense matrix would have 3-0 connectivity. A material having pores that pass completely through the matrix in one dimension would yield 3-1 connectivity, and a material having pores that interconnect two perpendicular faces but not the third would have 3-2 connectivity.

In a preferred embodiment, particularly for medical applications, the sizes of the interstices formed by the framework preferably are at least about 50 µm and preferably are on the order of 200 µm to about 600 µm. It is preferred that there be substantially no interstices less than 50 µm. In general, it is believed that in order to adequately support the growth of bone into the interstices, they must be capable of accommodating the passage of tissue having transverse dimensions of at least about 50 µm. Conceptually, it is convenient to think of a 50 µm interstice in materials of the invention as being capable of accommodating the passage through it of a "worm" having a round cross section and a transverse diameter of 50 µm. Put another way, a 50 µm interstice should enable passage through it of a sphere having a 50 µm diameter or smaller.

For medical applications, osteoconductive and osteoinductive materials can be included with both the porous and dense elements. The osteoconductive and osteoinductive materials that are appropriate for use in the present invention are biologically acceptable and include such osteoconductive materials as collagen and the various forms of calcium phosphates including hydroxyapatite; tricalcium phosphate; and fluoroapatite, bioactive glasses, osteoconductive cements, and compositions containing calcium sulfate or calcium carbonate, and such osteoinductive substances as: bone morphogenetic proteins (e.g., rhBMP-2); demineralized bone matrix; transforming growth factors (e.g., TGF-β); osteoblast cells, and various other organic species known to induce bone formation. Osteoinductive materials such as BMP may be applied to the combination dense/porous structure or the dense and/or porous elements individually, for example, by immersing the article in an aqueous solution of this material in a dilute suspension of type I collagen. Osteoinductive materials such as TGF-β may be applied to the combination dense/porous structure or the dense and/or porous elements individually from a solution containing an effective concentration of TGF-β. Cells capable of inducing bone formation such as osteoblasts, osteoblast precursors, mesenchymal stem cells, or marrow-derived stems cells may be suspended in an appropriate matrix such as a collagen gel and infiltrated into the interstices of the porous ceramic element of the article by means known in the art. Cells may also be cultured directly onto the surface(s) of the combination dense/porous structure or the dense and/or porous elements individually. Genetic material capable of inducing cells for forming bone may be applied to the porous element prior to implantation, directly applying a vector, such as an adenovirus, containing the genetic material to the combination dense/porous structure or the dense and/or porous elements individually with the intention of transfecting cells at the site of implantation. Alternatively, an appropriate cell type or types may be removed from the body, transfected *ex-vivo* and the cells applied to the combination dense/porous structure or the dense and/or porous elements individually prior to implantation.

Examples of these types of porous elements are described in U.S. Patent Nos. 6,136,029 and 6,296,667, owned by the assignee of the present application .

The porous element can be formed by methods known in the art. For example, in one preferred embodiment, a slip of ceramic material is made by combining a ceramic powder such as alumina with an organic binder and a solvent, such as water to form a dispersion or slip. The slip can also include other conventional additives such as dispersants, surfactants and defoamers. The strut surfaces of an organic reticulated foam such as one of the various commercially available foams made of polyurethane, polyester, polyether, or the like are wetted and coated with the ceramic slip. The reticulated material may be immersed in the slip, and then removed and drained to remove excess slip. If desired, further excess slip can be removed by any of a variety of methods including passing the material between a pair of closely spaced rollers or by impacting the material with a jet of air. Varying the slip concentration, viscosity, and surface tension provides control over the amount of slip that is retained on the foam strut surfaces. Wetting agents and viscosity control agents also may be used for this purpose. A wide variety of reticulated, open cell materials can be employed, including natural and synthetic materials and woven and non-woven materials, it being necessary in this embodiment only that the open cell material enables ceramic slip material to penetrate substantially fully through the interstices in the structure. According to a preferred aspect of making the porous element, a porous element can be provided that has one or more of: a greater degree of openness and connectedness than is possible with known methods; very fine porosities greater than those possible with known methods; and multiple layers of the same or different material. made possible by a multiple coating method with drying between each coating, such as described in US 6,977,095 , filed November 15, 1999 entitled "Process For Producing Rigid Reticulated Articles" . Specifically, after the coated substrate is dried after the first contacting with the dispersion, the coated substrate is then contacted with a second dispersion which can be the same or a different composition. After contacting with the second dispersion, the excess second dispersion is then removed and the coated substrate is dried as described above. This can be repeated with additional coatings of dispersions. What then results is a substrate having greater than one, and preferably 2 to 6 coatings. The use of multiple coatings is made possible by the use of the process described in US 6,977,095.

Once the reticular struts are coated with slip, the slip solvent is removed by drying, accompanied desirably by mild heating to form the green porous element. At this point, the green porous element can be used in its green state in the formation of the dense/porous structure. Alternatively, if the porous element is to be joined with a sintered dense element, as described more fully below, the porous element is first sintered by raising it to sintering temperatures at which the ceramic particles sinter to one another to form a rigid, light framework structure that mimics the configuration of the reticular struts. Before reaching sintering temperatures, the slip-treated reticulated, open cell material desirably is held at a temperature at which the organic material pyrolyzes or burns away, leaving behind an incompletely sintered ceramic framework structure which then is raised to the appropriate sintering temperature.

Pyrolyzing or oxidizing temperatures for most organics are in the range of about 200°C to about 600°C. Sintering temperatures for most ceramics of relevance to this invention are in the range of about 1100°C to about 1600°C, and preferred sintering temperatures for metals are in the range of about 800 to about 1400°C in a controlled atmosphere or in vacuum to prevent metals from oxidation.

Metals can be formed into frameworks, preferably hard, strong, continuous, and supportive, by a variety of manufacturing procedures including combustion synthesis, plating onto a "foam" substrate, chemical vapor deposition (see U.S. patent 5,282,861), lost mold techniques (see U.S. patent 3,616,841), foaming molten metal (see U.S. patents 5,281,251, 3,816,952 and 3,790,365) and replication of reticulated polymeric foams with a slurry of metal powder as described for ceramic powders.

The dense element of the combination dense/porous structure is a ceramic or metal and has a minimum sintered density of at least 95 %, preferably 97 % or 98 %. Suitable materials can be of the same or different from the porous element. A preferred material is a bio-inert ceramic such as zirconia or alumina or a bioactive ceramic such as hydroxyapatite, or tricalcium phosphate.

The dense element can be formed separately from the porous element and subsequently joined according to one aspect of the invention described in greater depth below. According to another aspect of the invention, the dense element is formed integrally with an already formed green porous element also described more in-depth below. In either case, when the material is a ceramic, the dense element is formed from a ceramic dispersion, such as a feedstock in injection molding or a ceramic slip for other applications such as slip casting formed into the desired shape and subsequently sintered according to methods known in the art.

The combination dense/porous structure also includes an interconnection zone that is defined as a portion of the combination that includes the interstices of at least a portion of the porous element being substantially filled, preferably completely filled with a material that also contacts at least a portion of the dense element. Preferably, the interconnection zone results from penetration of a fluent material (which material may be formed from the same or different material of the dense or porous element) into the porous element and contacting the dense element with the fluent material such that a strong bond is formed between the dense and porous element. As indicated above, the material that fills the interstices of at least a portion of the porous element can be the same or different from the material of the porous element or the dense element. In a preferred embodiment, the material is a ceramic and is the same as the dense element.

The processes of the present invention provide the ability to make custom dense/porous structures, preferably ceramic components requiring a multiplex structure having porous and dense elements, preferably with a uniform, consistent material make-up. Both the porous and dense elements are bonded together through the interconnection zone.

The formed combination dense/porous structure is useful in many applications, such as bone substitutes. In particular, one useful application is where a porous structure alone is insufficient to withstand physiological loading but host tissue ingrowth is desired. For example, a dense/porous ceramic construct could be designed for use in spinal fusion such that the dense elements bear the weight of the spinal column while the porous elements encourage bone to grow through the implant by osteoconduction, ultimately forming a bony bridge across the vertebra.

The dense portion also has the added advantage of confining the physiologically active agent that may be added to the porous portion as described above and releasing it in only preferred direction(s). By providing such directional control, bone growth occurs only in desired areas.

Other applications, described more fully below in connection with another aspect of the invention, include applications in which it is desired to mimic the structure of a bone that has both cortical (dense) and cancellous (porous) elements. For example, in a case in which a segment of a long bone is removed because of trauma or disease, the entire segment could be replaced by a dense/porous cylindrical object with a dense periphery to engage the cortical bone and bear the majority of the load while a porous center would contact the cancellous bone and bone marrow at either end. This would have the further advantage of encouraging host bone formation through the porous center of the implant, locking the implant into place and restoring blood flow through the center of the bone.

Another application would be in maxillofacial surgery in which restoration of contour is as important as restoration of function. In this case, the dense surface of a dense/porous combination structure could be formed in such a manner as to restore the contour of a bone, e.g., a portion of the facial skeleton following removal for trauma or disease, while a porous backing could encourage host bone in-growth, thereby integrating the combination into the host bone structure. In such an application, it may be preferred to use a bio-inert material for the dense portion, such as alumina or zirconia, to prevent the dense portion from being resorbed by natural bone, and the resulting natural bone being somewhat misshapen.

One method for producing the combination dense/porous structure described above, is to first provide a porous element in a green state. A first dispersion is made of a ceramic or metal powder, a binder and other optional known additives to form a feedstock for injection molding, or of a ceramic or metal powder, a binder, a solvent and optionally other known additives to form a slip for casting techniques. The dispersion is contacted with the porous element whereby the dispersion at least partially penetrates into at least a portion of the porous element to form an interconnection zone. The dense element is formed from the dispersion and adjacent to the interconnection zone to form a dense/porous structure. This structure is dried to form a green dense/porous structure. Sintering is then effected to form the combination dense/porous structure.

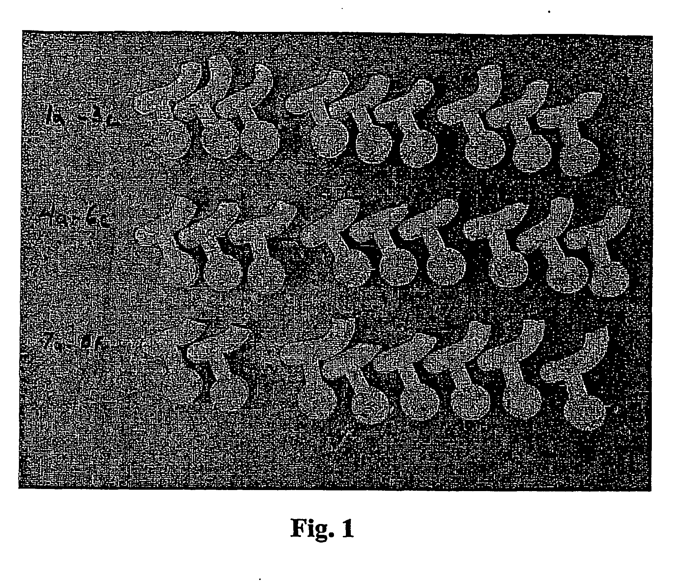

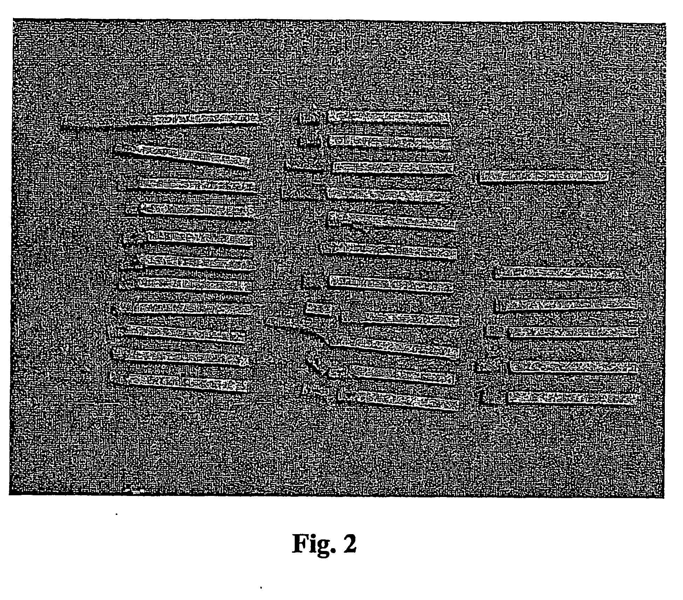

According to one preferred method, the combination dense porous structure can be formed by loading a green porous element into a mold, closing the mold, and injecting a feedstock into the mold to infiltrate, surround, or otherwise mate with the porous element in a process similar to insert molding to form the dense/porous structure that has the interconnection zone. Specifically, a green porous element is first produced as described above, such as from a foam. If not already shaped, the green ceramic foam may be shaped, such as by cutting. The shaped foam is then placed into the die (also called the "tool") of an injection molding machine. A ceramic feedstock, which will form the dense ceramic, is then injected into the tool. The feedstock is forced into the green ceramic foam as the cavity pressure rises, which forces the feedstock into the green porous body to form the interconnection zone. The feedstock in the cavity that is not forced into the green ceramic foam forms the green dense element, whose dimensions are formed by the tool and the green ceramic foam, to form a dense/porous structure. After removal of the green dense/porous combination from the tool, the green dense/porous structure is optionally debound, such as by heating or chemically, such as by a solvent. Sintering is then effected. The temperature for sintering is preferably in the range of about 1100°C to about 1600°C for ceramics, and preferably in the range of about 800 to about 1400°C for metals. In a preferred embodiment, at least one of the dimensions of the mold is larger than the porous element. This allows for formation of the dense element, without necessarily having to compress the porous element. Figures 1 and 2 show dense/porous structures that have been formed according to the injection molding embodiment of the present invention.

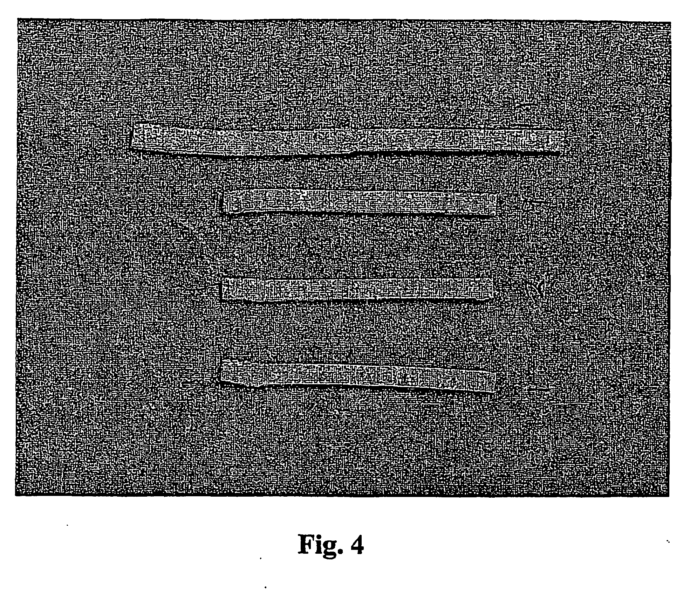

In one embodiment of the injection molding aspect of the present invention, the green ceramic foam compresses as cavity pressure rises. In this embodiment, the tool can have the same dimensions of the foam. This causes the foam portion to take up a smaller portion of the tool, and the dense portion to take up a larger portion of the tool. Upon debinding of the green dense/porous combination after it has been removed from the tool, the porous portion is restored to substantially its original dimensions and thus results in at least one dimension of the combination being larger than the corresponding dimension of the tool. This novel aspect can be very useful in that a smaller (and less expensive tool) can be used to make a larger combination dense/porous structure. See Figures 2 and 4 showing elements where the porous element has been compressed during injection molding.

If compression is not desired, such as in cases where compression would result in detrimental permanent distortion of the porous structure of the porous element, the interstices of the green porous element can first be filled with a hardenable and removable material such as a wax. The presence of the material, such as wax, in at least part of the porous element prevents compression of the element and thus modification of the porous structure. After injection, the material is removed by methods known in the art, such as heat or solvent. Debinding and sintering may then be carried out in the normal fashion. The result is a combination dense/porous structure with dimensions that correspond to the interior of the tool used to produce the structure.

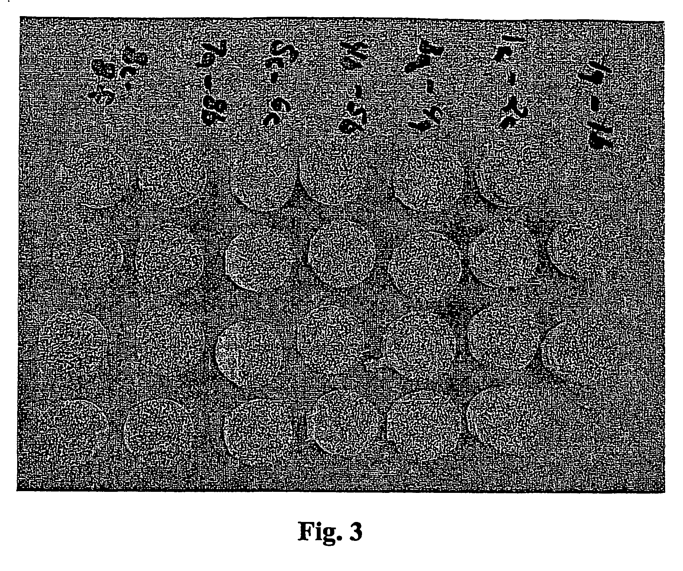

Another preferred embodiment provides for channels within the tool/porous structure to accommodate the feedstock flow front as the tool cavity is being filled. The channels can be entirely in the green porous structure, or alternatively can be bounded by a combination of the green porous element and the tool. In this embodiment, as the feedstock is injected into the tool, the feedstock will form a flow front that fills the tool. The feedstock flow front can be directed into the channels to transfer feedstock from the area of injection to another area of the tool. This results in being able to change the position of the dense and porous elements within the tool. Also, channeling some of the feedstock away from the injection site, reduces the compression of the dense element in that section, which may be preferred in some embodiments. Figure 3 shows dense/porous structures where channels have been formed in the green porous element.

According to another aspect of making the combination dense/porous structure, the green dense element is formed by a slip casting method. Specifically, a slip of a ceramic or metal is produced according to the method described above. The slip is poured into an open topped mold. Prior to the slip completely solidifying, a green porous ceramic element is placed into the slip, allowing the slip to partially or completely penetrate a portion of the porous element to form a dense/porous structure. The dense/porous structure is then dried and sintered to form the combination dense/porous structure.

Preferably, the mold is a porous mold that allows the liquid of the slip to be removed through capillary action. The mold has a cavity or an impression of the desired shape to form a ceramic shell. The liquid vehicle in the slip inside the mold is gradually removed through the capillary action of the pores of the plaster mold. A shell having the dimensions of the cavity or the impression begins to build up through this process, forming the green dense element. The dimensions such as thickness of the dense element can be controlled by topping the amount of slip in the mold over a time period. This is known in the field of fabricating ceramic components by slip casting technique for making dense ceramic articles such as sanitary ceramic ware. Once the desired dimensions (e.g., thickness) of the dense element are obtained, excessive slip may be drained. The dense element remains inside the mold having a wet surface exposed. Alternatively, if the dense element is allowed to dry, additional slip can be provided to re-wet the surface of the dense element that will contact the porous element. A green porous ceramic element is then seated and attached onto the still wet surface of the dense element. Preferably, the dense/porous structure is kept inside the mold allowing the residual moisture slowly to dry in a controlled manner. Due to the drying shrinkage, the dense/porous structure is readily separated from the mold and removed from the mold. Sintering can be carried out as described above.

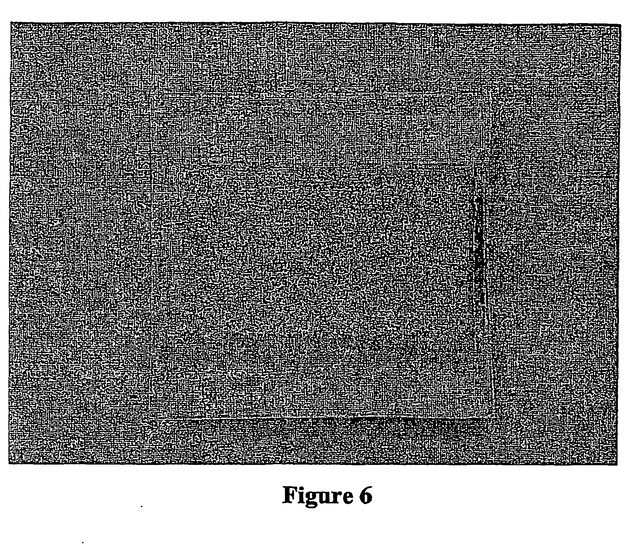

This slip casting embodiment provides a way of making custom dense/porous structures, preferably ceramic components requiring a multiplex structure having porous and dense elements. In this embodiment, the multiplex structure article can be produced at an especially reasonable cost. Intriguing features and shapes can be made by a sophisticated mold making craft. Figure 6 shows a dense/porous structure made according to this aspect of the invention.

According to another aspect of making the combination dense/porous structure, a sintered porous element is first prepared such as described above. A metal or ceramic dense sintered element is prepared separately. The sintered dense element and the sintered porous element are then joined using a bonding phase to form the interconnection zone. The bonding phase can include glass, ceramic, salts, inorganic polymers, organic polymers, metals, etc. In a preferred embodiment, a first dispersion of a ceramic or metal powder, a binder, and a solvent is combined to form a slip. The slip is applied to at least one surface of the dense or porous element, where the dense and porous elements are to be joined. A sufficient amount of slip is provided to allow the slip to penetrate into the porous element. The elements are joined and dried, forming a dense/porous structure having a green interconnection zone. The dense/porous structure is then subjected to heat for the bonding phase to develop sufficient strength to join the elements. Localized heating can be applied only to the bonding phase of the green interconnection zone to achieve such bonding strength using a laser beam and including other methods known in the art of material joining. A significant advantage of this method over prior art methods that typically join dense and porous elements in their green state is that stresses and defects that occur due to differential shrinkage during sintering are avoided. One application of this method is where a dense element and a porous element are fit together with a coating of slip between the dense and porous element. An example of a part produced using this process would have pillars of dense ceramic embedded in a block of porous ceramic. Another example is a thin walled cylinder of dense ceramic surrounding a central core of porous ceramic.

To control or limit the penetration of the material forming the dense element into the porous element, or to control the penetration of the bonding phase material or slip used to combine sintered dense and porous elements into the porous element, a component that fills part or all of the porosity of the porous element may be used. Subsequent to combining these elements, this component may removed from the porous element by mechanical, thermal, non-aqueous solvent, water, catalytic, sublimation, enzymatic, acid, base or other means of destruction or combinations thereof which destructively disengages the additional component without substantially damaging the dense/porous structure while restoring the porous nature of the porous portion of the dense/porous combination. "Substantially without damaging the molded object" is defined as the dense/porous combination not being degraded mechanically and preferably having no more than minor cosmetic surface flaws, e.g., scratches. Preferably the object has no surface flaws. Examples of a third component include but are not limited to glass, ceramics, salts, inorganic polymers, organic polymers, metals, etc. This component can include the hardenable material described above to prevent compression during injection molding. Likewise, the hardenable material can include the materials of this component.

According to another aspect of the invention, there has been provided a combination dense/porous structure useful as a bone substitute. Prior to the present invention, bone substitutes were typically used only to replace relatively small sections of a bone and were generally not weight bearing. However, a dense/porous combination makes it possible to include within the bone substitute a structure that is analogous to natural bone. In one embodiment, the bone substitute includes the combination dense/porous structure described above. Preferably, the porous element of the combination dense/porous structure mimics the cancellous structure of natural bone and the dense element mimics the cortical structure of the bone

In another embodiment, the bone substitute has a shape that substantially corresponds to an entire cross-section of bone. In addition, the bone substitute may also have another dimension such as a length that substantially corresponds to the length of the natural bone it is replacing. In a preferred embodiment, the bone being replaced is a "long bone," which term in known in the art. Particularly, a long bone is a bone such as a femur or tibia, that includes a freely movable or slightly movable joint at one or both ends.

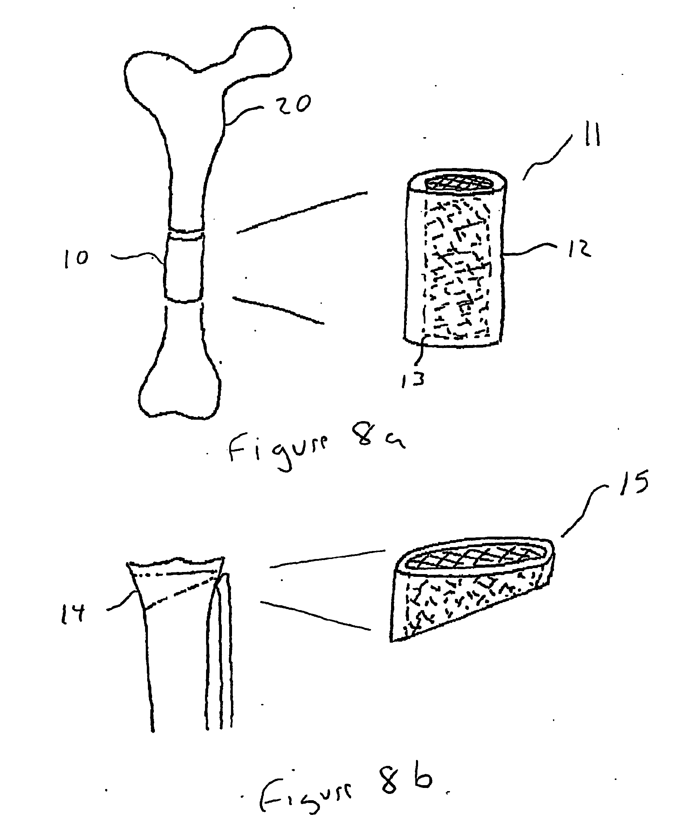

Figure 8a describes one embodiment of the invention in which a segment of the diaphysis 10 of the femur 20 is replaced by an artificial bone structure 11 containing dense 12 and porous 13 elements that substantially correspond to the cortical and cancellous bone elements that are being removed. This embodiment is particularly useful in situations where a segment of bone is irreparably damaged due to traumatic injury, infection, tumor, or other disease. This invention may also be used similarly to replace a segment of the metaphysis 14 of a bone, for example, in the correction of an angular deformity of the tibia 15 as illustrated in Figure 8b with artificial bone structure 15. These examples are non-inclusive and only illustrate representative applications of the invention.

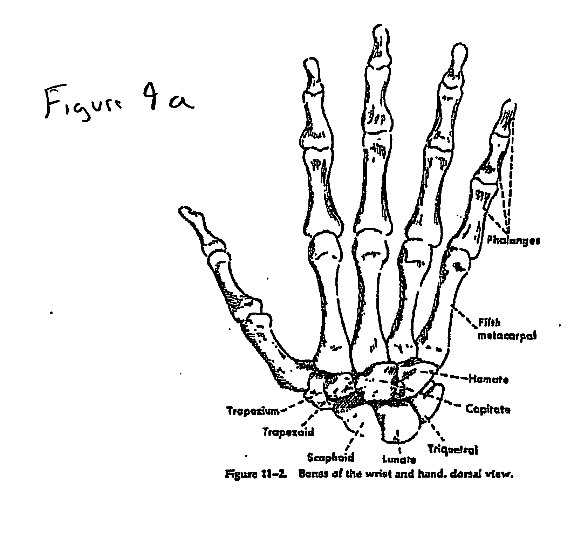

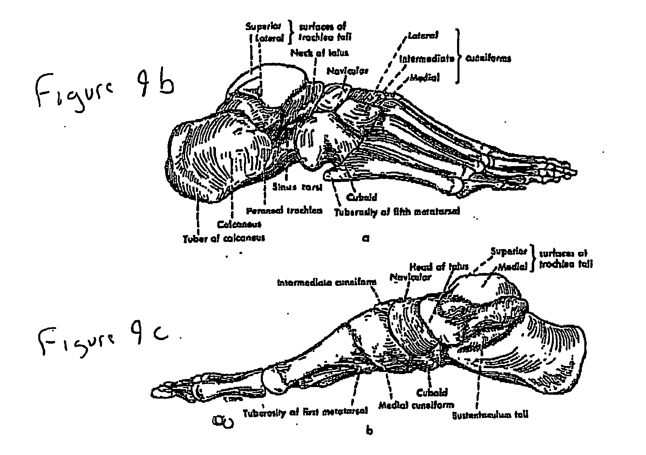

The artificial bone structure of this invention may also be used to substantially replace a bone in its entirety. This would be particularly appropriate in the smaller bones of the extremities particularly the carpal, metacarpal, phalangeal, tarsal, and metatarsal bones as illustrated in Figures 9a-9c, the vertebral bodies, and the bones of the facial skeleton. However, the invention is scalable and one skilled in the art could apply the invention to the replacement of larger bones.

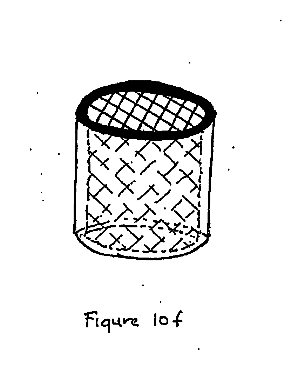

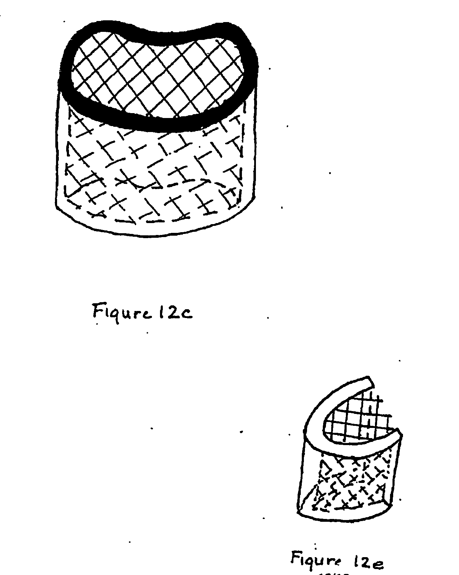

The artificial bone structure includes an inner porous portion formed from a porous element to mimic the cancellous structure of bone. This porous element may be the same or different from the porous element described above with respect to the combination dense/porous structures. Preferably, the porous element is the same as the porous element described above. An outer dense portion completely surrounding the inner porous portion formed from a dense element to mimic the cortical structure of bone. This dense element may be the same or different from the dense element described above with respect to the combination dense/porous structures. Figure 10f shows an example of this embodiment and Figure 12c shows an artificial bone structure having a structure similar to Figure 10f.

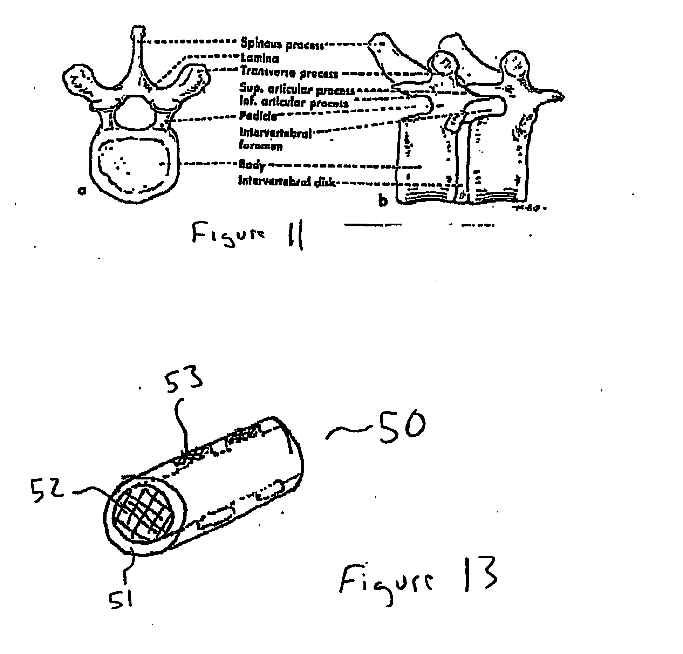

In another embodiment, an artificial bone structure (Figure 12e) may be created in which the dense element(s) partially surround the porous element in order to facilitate the fusion of two vertebrae, particularly in the cervical region of the spinal column. In this embodiment, the dense element(s) bear part of the load through the spinal column while the porous element facilitates bone ingrowth through the artificial bone structure and fusion of the vertebrae. In another embodiment (50, Figure 13), an artificial bone structure may be created in which the dense element surrounds the porous element and contains holes that expose the porous element to the outer surface of the dense element. This will encourage bone ingrowth in three dimensions into the artificial bone structure. In one embodiment illustrated in Figure 13, the dense element 51 is substantially cylindrical with holes 53 oriented perpendicular to the longitudinal axis of cylinder. The inner porous element 52 is exposed at the longitudinal ends of the cylinder and through the holes. In this embodiment, the artificial bone structure may be placed between two vertebrae with the longitudinal axis of the cylinder oriented in the transverse anatomic plane and the holes in contact with the vertebrae in order to facilitate bone ingrowth through the porous element, thereby fusing the vertebrae, while the dense element supports the weight of the spinal column.

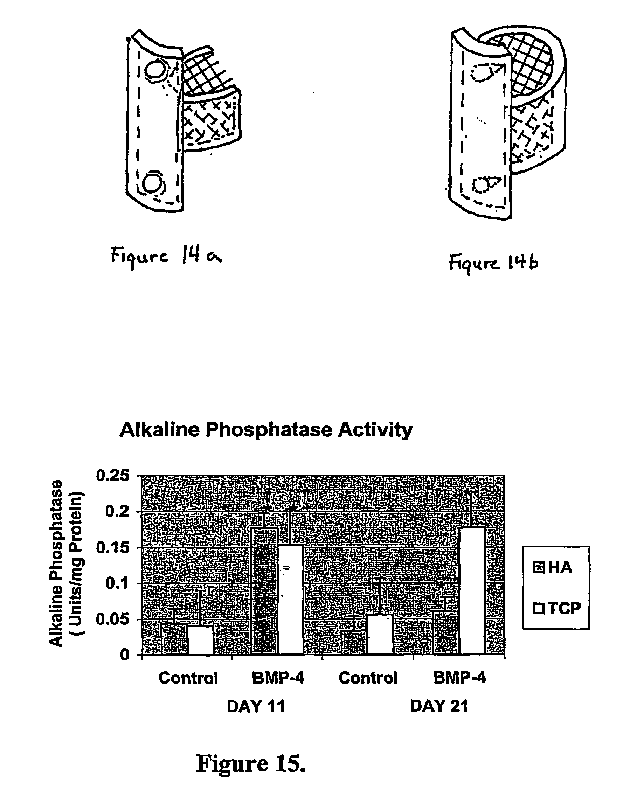

In a further embodiment, an artificial bone structure (Figure 14a, b) may be created in which the dense element(s) partially or completely surround the porous element and the dense element(s) are used to stabilize the porous element. In this embodiment, the dense element may be designed in such as way as to fix itself to the surrounding structures, e.g., by means of spikes, or the dense element may be designed to accept a third component, e.g., a screw, to fix the dense element to the surrounding structures, thereby fixing the porous element in place and facilitating bone ingrowth through the artificial bone structure.

Another aspect of the invention provides a delivery system, such as for a physiologically active agent, preferably a sustained release delivery system. In this aspect of the invention, the porous element contains the physiologically active agent, and the dense element surrounds at least a portion of the porous element. Physiologically active agents may include but are not limited to autologous cells, exogenous cells, chemical signals (including growth factors), genetic material, or naturally derived or synthetically produced pharmaceuticals and combinations thereof. These agents may also include substances such as bone marrow, blood, plasma, demineralized bone matrix, or morsellized bone of the patient or from a suitable donor. The bone marrow, blood, plasma, demineralized bone matrix, or morsellized bone may have been minimally processed before being introduced into the porous element of the invention or it may have been significantly modified while outside the body, for example, by filtering, heating, or sterilizing. In a preferred embodiment, the agent is an osteoinductive material, which may include but is not limited to bone morphogenetic protein, members of the Transforming Growth Factor Beta superfamily of molecules, osteoblast cells, mesenchymal stem cells, or various other organic species known to induce bone formation. In a most preferred embodiment, the agent is recombinant human Bone Morphogenetic Protein-4 (rhBMP-4).

Due to the interconnected interstices of the porous element, the physiologically active agent is able to be loaded into the system at a therapeutically effective amount, preferably to be released over an extended period of time, on the order of 24 hours, more preferably 2 days, more preferably 5 days, 7 days, 2 weeks, one month, or even longer. The dense and porous elements are preferably made from a ceramic. In another preferred embodiment, the dense and porous elements are the combination dense/porous structure described above.

The delivery system can be implanted in an suitable area of the body, as long as the active agent, when released, is able to be delivered, such as by the blood stream, to the area of the body in need of the active agent. Preferred areas of implantation would include bone, subcutaneous, and intramuscular sites. In some embodiments, it would not be necessary to even implant the delivery system. For example, a patient could swallow or have the delivery system delivered to the stomach orally. Likewise, the delivery system could be delivered as a suppository.

In one preferred embodiment, the delivery of the active agent from the delivery device is controlled by the amount of dense element that surrounds the porous element. In another preferred embodiment, the dense element is a bioresorbable material and the delivery of the active agent from the porous element is controlled by the dissolution of the dense element. In another preferred embodiment, the direction of release of the physiologically active agent can be controlled by the placement of the dense element in a similar manner as described above with respect to the release of the physiologically active agent. For example, delivering an osteoinductive agent, such as BMP, when the combination dense/porous structure is being used as a bone substitute.

The delivery device can be prepared by any of the methods described herein, in addition to any other suitable method.

The present invention may be more easily understood by reference to the following non-limiting examples:

### Example 1

Tricalcium Phosphate (TCP) feedstock was prepared by combining the following ingredients:
Polypropylene: 48.47 grams
Polyethylene: 17.23 grams
Paraffin wax: 37.7 grams
Stearic acid: 5.09 grams
TCP powder: 586 grams

A bar tool was used to mold the TCP. The tool cavity measured 0.1905" by 0.14" by 2.35 long. Green TCP porous elements were cut into blocks that were 0.1905" by 0.14" with lengths from 25% to 85 % of cavity length. TCP feedstock was used for the dense portion.

After a stable cycle was created on the injection molding machine, one block per cycle was placed in the cavity prior to filling the tool with the TCP feedstock. Different locations for porous material placement were carried out with particularly good results occurring in the samples where the material was placed at the last point to fill. This resulted in the material meeting and integrating across the bar's smaller dimensions.

In some cycles, unaltered green porous material was used. In other instances, a wax was introduced into the porous structure to fill the interstices prior to placement into the tool. The wax was used to reduce the compressibility of the porous material during the filling of the tool with dense material. Other substances, such as salt, could be substituted for the wax.

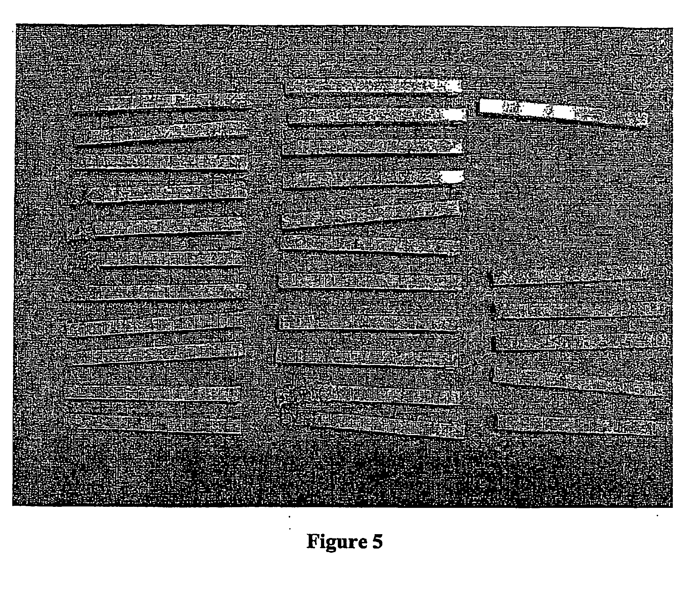

When the molded articles were removed from the tool the green porous ceramic had compressed to approximately 25% of its original length. See Figure 5. The green porous ceramic, which had been infiltrated with wax, compressed significantly less.

The parts were then placed in a solvent to remove the wax content of the molded article. During this process, the porous ceramic portion of the molded article was substantially restored to its original shape and size. See Figure 2. The parts were then sintered as normal resulting in the finished articles pictured as shown in Figure 4. In another example, a disc shape cavity was used for a tool. It measured 0.25" thick by 0.5" diameter.

Green porous foam was cut to match the diameter with thicknesses approximately one-third of and equal to the full cavity thickness. The green porous material as well as the feedstock were both comprised of alumina from the following composition:
Polypropylene: 48.47 grams
Polyethylene: 17.23 grams
Paraffin wax: 37.7 grams
Stearic acid: 5.09 grams
Alumina powder: 586 grams.

In the porous foam whose thickness matched the cavity thickness, a channel was cut 0.25" thick by 0.25" deep across the face of the diameter. This took advantage of the fill pattern of the tool by allowing the flow front of the feedstock to fill the channel first then filling the thickness second causing the porous material to be compressed uniformly across it's thickness, as shown in Figure 1. As in the prior example, the foam compressed to approximately 25% of its original thickness and restored its original shape during solvent debind. See Figure 3.

### Example 2

A combination dense/porous structure of ceramic materials was made as follows: A ceramic slip was created using 58% hydroxyapatite powder, 17% water, 22.5% acrylic binder, 2% dispersant, 0.4% surfactant, and 0.4% defoamer, based on the entire weight of the slip. The slip was then used to coat a reticulated foam structure. Excess slip was then removed to allow only the struts of the foam to be coated and to allow an interconnected structure. The same slip was then used to "paint" the desired surfaces of the foam. Several coats were used to build up the required thickness. After drying the samples were sintered to remove the organic material and densify the ceramic.

### Example 3

A reticulated porous element was prepared as mentioned above. Rather than coat the edges, the green reticulated porous element is then fired to densify the ceramic. At the same time, a dense element is prepared by a preferred method, this may be by injection molding, slip casting, etc. After the two pieces are both densified they are combined with a bonding phase. The material(s) of this bonding phase may be another ceramic slip, glass, etc. This phase is used to treat the appropriate surfaces to be bonded and is then cured using a secondary sintering or other curing processes.

### Example 4

A batch of slip was prepared by mixing 58 % hydroxyapatite powder, 17 % water, 22.5% acrylic binder, 2 % dispersant, 0.4% surfactant, and 0.4% defoamer, based on the entire weight of the slip. Polyurethane foam was used as a precursor to fabricate the portion of porous structure. The green porous element was then prepared as described above.

The same slip was poured into a plaster mold having a two-inch square cavity with a 0.5 inch depth. After 15 minutes excessive slip was drained out, A dried porous element having dimensions of 2 inches square by 0.5 inches thick was inserted into the same plaster mold. A light pressure was applied to the top surface to ensure that the residual slip penetrates into the porous element where the slip cast green dense element in contact with the porous element to form an interconnection zone. The combination of the green dense/porous structure was left in the plaster mold dried for at least two hours.

The dried combination of dense/porous structure was then placed in a kiln to burn out the binder and polymer precursor, subsequently sintered at a temperature of 1300°C to densify the combination dense/porous structure.

The following example describes delivery of rhBMP-4 from a porous ceramic carrier. This experiment did not use a dense-porous structure but is included for illustrative purposes only to show how the physiologically active agent can be introduced into the porous element and can be delivered *in vivo.* The mechanism of action described in this example is identical to that which would be expected using a combination dense/porous structure.

### Example 5

Reticulated porous elements were prepared as described in Example 4 from tricalcium phosphate (TCP) and hydroxyapatite (HA). Implants were cut into discs while in the green state following drying. The coated foams were sintered between 1200 and 1550°C for 2 to 10 hours, depending on the composition. The sintered discs had a diameter of 8.5 mm and a thickness of 2 mm. Implants were placed subcutaneously in 28-35 day old Long-Evans rats. The implants were either implanted alone (control) or with a dose of 3 µg of rhBMP-4 (R&D Systems, Minneapolis, MN) reconstituted in sterile saline adsorbed into the porous ceramic. The implants were air-dried in a hood prior to implantation.

The animals were sacrificed at 11 and 21 days and the concentration of alkaline phosphatase, a biochemical marker for new bone formation was measured. A limited number of implants were reserved for histology and fixed in formalin, demineralized, and sections cut and stained with 0.1 % toluidine blue.

Porous ceramic implants alone in this heterotopic subcutaneous site were not as osteogenic at these time points. However, when combined with 3 µg of rhBMP-4, the HA and TCP implants were significantly more osteogenic. The osteogenic effect observed with HA+BMP implants was maximal on Day 11, then declined by Day 21, although it remained higher than in control HA implants. The osteogenic effect with TCP+BMP implants was elevated on both Day 11 and Day 21 with no significant change in activity level, indicating a sustained osteogenic response. All implants were welltolerated and biocompatible, with vascularized soft tissue invading and filling the interstices of control implants. No significant evidence of new bone formation was found in implants without BMP in this particular model. In HA and TCP implants with BMP, new bone formation was found throughout the implant at Days 11 and 21, mirroring the biochemistry data. These data are summarized in Figure 15.

## Claims

1. A combination dense/porous structure used as a bone substitute, comprising: a porous element having an outer surface defining a shape having a bulk volume, the element comprising a continuous framework having struts defining a plurality of interconnecting interstices, throughout the bulk volume, said framework having a reticulated structure with 3-3 connectivity, said porous element having interconnecting interstices extending throughout said volume and opening through said surface, and the material of said porous element comprising ceramics, metals and combinations thereof; a ceramic or metal dense element formed from a material and having a sintered density of at least 95 % contacting at least a portion of the porous element; a bonding phase; and an interconnection zone joining at least one surface of the porous element and dense element whereby the bonding phase penetrates into the porous element.

2. A combination dense/porous structure according to claim 1, wherein the average interstices are wider than the thicknesses of the struts.

3. A combination dense/porous structure according to claim 1, comprising an interconnection zone formed by the inter-penetration of the material forming the dense element into the porous element.

4. A combination dense/porous structure according to claim 3, wherein the bulk volume has an inward facing surface and the dense portion contacts at least a portion of the inward facing surface.

5. A combination dense/porous structure according to claim 3, wherein the porous and dense elements comprise a ceramic.

6. A combination dense/porous structure according to claim 3, wherein the porous element has a porosity of 80% or greater.

7. A combination dense/porous structure according to claim 3, wherein the average interstices are wider than the thicknesses of the struts.

8. A combination dense/porous structure according to claim 3 formed by a process comprising: providing a porous element in a green state; providing a first dispersion of a ceramic or metal powder, a binder, and a solvent to form a slip; contacting a slip with at least a portion of the porous element whereby the slip at least partially penetrates into at least a portion of the porous element to form an interconnection zone and a dense element adjacent to the interconnection zone; sintering the combination dense/porous structure.

9. A combination dense/porous structure according to claim 8, wherein the porous and dense elements comprise a ceramic.

10. A combination dense/porous structure useful as an artificial bone structure comprising an element having a shape of natural bone or a portion of natural bone and a cross-section that comprises the combination dense/porous structure according to claim 3 having an inner porous portion formed from the porous element to mimic the cancellous structure of bone; and an outer dense portion completely surrounding the inner porous portion formed from the dense element to mimic the cortical structure of bone.

11. A combination dense/porous structure useful as an artificial bone structure as claimed in claim 10 in the shape of a replacement for a segment of long bone.

12. A combination dense/porous structure useful as an artificial bone structure as claimed in claim 10 in the shape of the metaphyseal or diaphyseal segment of a bone.

13. A combination dense/porous structure useful as an artificial bone structure as claimed in claim 10 in the shape of an entire bone.

14. A combination dense/porous structure useful as an artificial bone structure as claimed in Claim 10 in the shape of a vertebral body.

15. A bone substitute material comprising a combination dense/porous structure according to claim 3.

16. A combination dense/porous structure according to claim 1, comprising: a sintered porous element.

17. A combination dense/porous structure according to claim 16, wherein the average interstices are wider than the thicknesses of the struts.

18. A combination dense/porous structure according to claim 16, wherein the combination dense/porous structure is produced by a process comprising: providing a sintered porous element; providing a sintered ceramic or metal dense element formed from a material and having a sintered density of at least 95 %; providing a bonding phase; joining at least one surface of the sintered porous element and the sintered dense element with the bonding phase, whereby the bonding phase penetrates into the porous element and forms an interconnection zone; and
(e) curing, drying, and/or heating, if necessary, the bonding phase to form the combination dense/porous structure.

19. A combination dense/porous structure according to claim 3, wherein the combination dense/porous structure is produced by a process, comprising:
(a) providing a porous element in a green state;
(b) providing a dispersion comprising a ceramic or metal powder and a binder;
(c) contacting the dispersion with the porous element whereby the slip at least partially penetrates into at least a portion of the porous element to form an interconnection zone and a dense element formed from the dispersion and adjacent to the interconnection zone to form a combination dense/porous structure;
(d) solidifying the combination dense/porous structure to form a green combination dense/porous structure; and
(e) sintering the green combination dense/porous structure to form the combination dense/porous structure.

20. A combination dense/porous structure according to claim 1 useful as a sustained release delivery system comprising: a reticulated, porous element having an open interconnected porosity; and a dense element surrounding at least a portion of the porous element, wherein at least one of the dense or porous element contains a physiologically active agent.

21. A sustained release delivery system according to claim 20, wherein the porous element contains the physiologically active agent.

22. A sustained release delivery system according to claim 20, wherein porous element has an outer surface defining a shape having a bulk volume, the element comprising a continuous framework having struts defining a plurality of interconnecting interstices throughout the bulk volume, and said porous element having interconnecting interstices extending throughout said volume and openings through said surface.

23. A sustained release delivery system according to claim 20, wherein the average interstices are wider than the thicknesses of the struts.

24. A sustained release delivery system according to claim 20, wherein the porous element and dense element comprise a ceramic.

25. A sustained release delivery system according to claim 21, wherein the dense element surrounds a portion of the porous element and the delivery of the physiologically active agent from the porous element is controlled by the thickness of dense element surrounding the porous element.

26. A sustained release delivery system according to claim 21, wherein the dense element surrounds a portion of the porous element and the delivery of the physiologically active agent from the porous element is controlled by the surface area of the porous element that is covered by the dense element.

27. A sustained release delivery system according to claim 21, wherein the dense element is a bioresorbable material and the delivery of the physiologically active agent from the porous element is controlled by the bioabsorption of the dense element.

28. A sustained release delivery system according to claim 24, wherein the combination dense/porous structure is produced by a process comprising: providing a porous element; providing a ceramic or metal dense element formed from a material and having a sintered density of at least 95 %; providing a bonding phase; joining at least one surface of the porous element and the dense element with the bonding phase, whereby the bonding phase penetrates into the porous element and forms an interconnection zone; drying the bonding phase to form the combination dense/porous structure; and sintering the combined dense and porous element with the interconnection zone to form the combination dense/porous structure.

29. A process for producing a combination dense/porous structure according to claim 1 comprising:
(a) providing a porous element having an outer surface defining a shape having a bulk volume, the element comprising a framework having struts defining a plurality of interconnecting interstices throughout the bulk volume, said framework having a reticulated structure with 3-3 connectivity, said element having interconnecting interstices extending throughout said volume and opening through said surface, and the material of said porous element comprising ceramics, metals and combinations thereof;
(b) providing a dense element formed from a material and having a sintered density of at least 95%;
(c) providing a bonding phase;
(d) joining at least one surface of the porous element and the dense element with the bonding phase, whereby the bonding phase penetrates into the porous element and forms an interconnection zone;
(e) drying the bonding phase to form the combination dense/porous structure; and
(f) sintering the combined dense and porous element with the interconnection zone to form the combination dense/porous structure.

30. A process for producing a combination dense/porous structure according to claim 29, wherein the bonding phase comprises a dispersion of a ceramic or metal powder, a binder, and a solvent in the form of a slip.

31. An injection molding process for producing a combination dense/porous structure according to claim 3 comprising:
(a) providing a porous element in a green state produced from an organic reticulated foam;
(b) inserting the porous element into a mold;
(c) providing a first dispersion comprising a ceramic or metal powder and a binder, form a feedstock;
(d) injection molding the feedstock into the tool for a time and pressure sufficient to force the feedstock into at least a portion of the porous element to form a molded green dense/porous structure;
(e) removing the molded green dense/porous structure from the tool;
(f) debinding the molded structure; and
(g) sintering the molded green combination dense/porous structure to form the combination dense/porous structure.

32. A process according to claim 31, wherein the tool cavity has at least one dimension larger than a corresponding dimension of the porous element.

33. A process according to claim 31, wherein the step of injection molding the feedstock compresses the porous green element into a volume smaller than the uncompressed volume of the porous element, and the step of debinding the molded green dense/porous structure expands the porous green element, such that the sintered dense/porous structure has a dimension larger than a corresponding dimension of the tool cavity.

34. A process according to claim 31, further comprising: filling at least a portion of the interstices of the green porous element with a material that maintains the shape of the green porous element during injection molding; and removing the material from the green porous element such that the dimensions of the green porous element remain substantially identical to the dimensions before injection molding.

35. A process according to claim 31, wherein the porous element in a green state includes channels for accommodating the feedstock flow front as the tool cavity is filled.

36. A process according to claim 34, wherein the material is a wax and the removal of the material from the green porous element comprises applying a solvent to the green porous element.

37. A process according to claim 34, wherein the material is a polymer and the removal of the material from the green porous element comprises applying a solvent to the green porous element.

38. A process according to claim 34, wherein the material is a salt and the removal of the material from the green porous element comprises applying a solvent to the green porous element.

39. A process according to claim 34, wherein the material is a wax and the removal of the material from the green porous element comprises applying heat to the green porous element.

40. A process according to claim 34, wherein the material is a polymer and the removal of the material from the green porous element comprises applying heat to the green porous element.

41. A process according to claim 34, wherein the material is a salt and the removal of the material from the green porous element comprises applying heat to the green porous element.

42. A slip casting process according to claim 29 for producing a combination dense/porous structure according to claim 3, comprising:
(a) providing a porous mold;
(b) providing a first dispersion of a ceramic or metal powder, a binder, and a solvent to form a slip;
(c) pouring the slip into the mold, whereby the solvent in the slip is removed through the mold by capillary action to form a green dense element;
(d) optionally adding additional slip to adjust one or more dimensions of the dense element;
(e) providing a green porous element;
(g) contacting the green porous element with the dense green element while at least one surface of the dense element is wet with slip;
(h) drying to form a green dense/porous structure;
(i) removing the green dense/porous structure; and
(j) sintering the dense/porous structure to form the combination dense/porous structure.

43. A process according to claim 42, further comprising removing excess slip from the mold when a desired dimension is reached and wherein the wet slip on the at least one surface of the dense element is from the slip used to form the solid element.

44. A process according to claim 42, further comprising adding additional slip to retain minimum wet slip for providing an interconnection zone between the porous and dense element.

45. A process according to claim 29 for producing a combination dense/porous structure according to claim 3, comprising:
(a) providing a porous element in a green state;
(b) providing a first dispersion of a ceramic or metal powder, a binder, and a solvent to form a slip;
(c) coating at least a portion of one surface of the porous element with the slip whereby the slip at least partially penetrates into at least a portion of the porous element to form the interconnection zone and a dense element is formed from the slip and adjacent to the interconnection zone to provide a dense/porous structure;
(d) optionally adding further coats of slip to the porous element;
(e) drying the combination dense/porous structure to form a green dense/porous structure; and
(f) sintering the green dense/porous structure to form a combination dense/porous structure.

46. A process according to claim 45, wherein the coating at least one surface comprises brushing the slip onto the at least one surface.

47. A process according to claim 29 for producing a combination dense/porous structure according to claim 21 comprising:
(a) providing a sintered porous element having an outer surface defining a shape having a bulk volume, the element comprising a framework having struts defining a plurality of interconnecting interstices throughout the bulk volume, said framework having a reticulated structure with 3-3 connectivity, said element having interconnecting interstices extending throughout said volume and opening through said surface, and the material of said porous element comprising ceramics, metals and combinations thereof;
(b) providing a sintered dense element formed from a material and having a sintered density of at least 95 %;
(c) providing a bonding phase;
(d) joining at least one surface of the sintered porous element and the sintered dense element with the bonding phase, whereby the bonding phase penetrates into the porous element and forms an inter-penetration zone; and
(e) curing, drying, and/or heating, if necessary, the bonding phase to form the combination dense/porous structure.

48. A process according to claim 47 for producing a combination dense/porous structure, wherein the bonding phase comprises a dispersion of a ceramic or metal powder, a binder, and a solvent in the form of a slip.

## Patentansprüche

1. Dichte/poröse Strukturkombination, die als Knochenersatz verwendet wird, umfassend: ein poröses Element mit einer äußeren Oberfläche, die eine Form mit einem Rohvolumen definiert, wobei das Element im gesamten Rohvolumen ein kontinuierliches Netzwerk mit Verstrebungen umfasst, die eine Vielzahl von verbindenden Zwischenräumen definieren, wobei dieses Netzwerk eine vernetzte Struktur mit 3-3-Konnektivität aufweist, wobei das poröse Element verbindende Zwischenräume aufweist, die sich über das gesamte Volumen erstreckt und sich durch diese Oberfläche hindurch öffnen; und wobei das Material des porösen Elements Keramiken, Metalle und Kombinationen davon umfasst; ein dichtes Keramik- oder Metallelement, das aus einem Material gebildet ist und eine Sinterdichte von mindestens 95 % aufweist und mit mindestens einem Teil des porösen Elements in Kontakt steht; eine klebende Phase und eine Verbindungszone, die mindestens eine Oberfläche des porösen Elements und des dichten Elements verbindet, wobei die klebende Phase in das poröse Element eindringt.

2. Dichte/poröse Strukturkombination nach Anspruch 1, wobei die mittleren Zwischenräume weiter sind als die Dicke der Verstrebungen.

3. Dichte/poröse Strukturkombination nach Anspruch 1, umfassend eine Verbindungszone, die durch das Eindringen des das dichte Element bildenden Materials in das poröse Element gebildet wird.

4. Dichte/poröse Strukturkombination nach Anspruch 3, wobei das Rohvolumen eine nach Innen gerichtete Oberfläche aufweist und der dichte Teil mit mindestens einem Teil der nach Innen gerichteten Oberfläche im Kontakt steht.

5. Dichte/poröse Strukturkombination nach Anspruch 3, wobei die porösen und dichten Elemente eine Keramik umfassen.

6. Dichte/poröse Strukturkombination nach Anspruch 3, wobei das poröse Element eine Porosität von mindestens 80 % aufweist.

7. Dichte/poröse Strukturkombination nach Anspruch 3, wobei die mittleren Zwischenräume weiter sind als die Dicke der Verstrebungen.

8. Dichte/poröse Strukturkombination nach Anspruch 3, gebildet durch ein Verfahren, umfassend: Bereitstellen eines porösen Elements in einem Grünzustand; Bereitstellen einer ersten Dispersion eines Keramik-oder eines Metallpulvers, eines Bindemittels und eines Lösungsmittels zur Bildung eines Schlickers, das In-Kontakt-Bringen eines Schlickers mit mindestens einem Teil des porösen Elements, wodurch der Schlickers zumindest teilweise in mindestens einen Teil des porösen Elements eindringt, um eine Verbindungszone und ein der Verbindungszone benachbartes dichtes Element zu bilden; Sintern der dichten/porösen Strukturkombination.

9. Dichte/poröse Strukturkombination nach Anspruch 8, wobei die porösen und dichten Elemente eine Keramik umfassen.

10. Dichte/poröse Strukturkombination, die als eine künstliche Knochenstruktur von Nutzen ist, umfassend ein Element mit einer Form eines natürlichen Knochens oder eines Teiles eines natürlichen Knochens und einem Querschnitt, der die dichte/poröse Strukturkombination nach Anspruch 3 mit einem inneren porösen Teil, der zur Nachahmung der Schwammstruktur des Knochens aus dem porösen Element gebildet wurde, und mit einem äußeren dichten Teil umfasst, der zur Nachahmung der Substantia kompakta des Knochens den inneren porösen Teil vollständig umgibt und der aus dem dichten Element gebildet wurde.

11. Dichte/poröse Strukturkombination, die als eine künstliche Knochenstruktur von Nutzen ist, nach Anspruch 10 in der Form eines Ersatzes für ein Segment eines Röhrenknochens.

12. Dichte/poröse Strukturkombination, die als eine künstliche Knochenstruktur von Nutzen ist, nach Anspruch 10 in der Form des Metaphysen- oder Diaphysensegments eines Knochens.

13. Dichte/poröse Strukturkombination, die als eine künstliche Knochenstruktur von Nutzen ist, nach Anspruch 10 in der Form eines ganzen Knochens.

14. Dichte/poröse Strukturkombination, die als eine künstliche Knochenstruktur von Nutzen ist, nach Anspruch 10 in der Form eines Vertebralkörpers.

15. Knochenersatzmaterial, umfassend eine dichte/poröse Strukturkombination nach Anspruch 3.

16. Dichte/poröse Strukturkombination nach Anspruch 1, umfassend: ein gesintertes poröses Element.

17. Dichte/poröse Strukturkombination nach Anspruch 16, wobei die mittleren Zwischenräume weiter sind als die Dicke der Verstrebungen.

18. Dichte/poröse Strukturkombination nach Anspruch 16, wobei die dichte/poröse Strukturkombination durch ein Verfahren erzeugt wird, umfassend: Bereitstellen eines gesinterten porösen Elements; Bereitstellen eines gesinterten dichten Keramik- oder Metallelements, das aus einem Material gebildet ist und eine Sinterdichte von mindestens 95 % aufweist; Bereitstellen einer klebenden Phase, Verbindung mindestens einer Oberfläche des gesinterten, porösen Elements und des gesinterten, dichten Elements mit der klebenden Phase, wodurch die klebende Phase in das poröse Element eindringt und eine Verbindungszone bildet; und
(e) gegebenenfalls Härten, Trocknen und/oder Erhitzen der klebenden Phase zur Bildung der dichten/porösen Strukturkombination.

19. Dichte/poröse Strukturkombination nach Anspruch 3, wobei die dichte/poröse Strukturkombination durch ein Verfahren erzeugt wird, umfassend:
(a) Bereitstellen eines porösen Elements in einem Grünzustand;
(b) Bereitstellen einer Dispersion, umfassend ein Keramik-oder ein Metallpulver und ein Bindemittel;
(c) In-Kontakt-Bringen der Dispersion mit dem porösen Element, wodurch der Schlicker zumindest teilweise in mindestens einen Teil des porösen Elements eindringt, um eine Verbindungszone zu bilden und sich dazu benachbart aus der Dispersion ein dichtes Element bildet, um eine dichte/poröse Strukturkombination zu bilden;
(d) Verfestigen der dichten/porösen Strukturkombination zur Bildung einer grünen dichten/porösen Strukturkombination; und
(e) Sintern der grünen dichten/porösen Strukturkombination zur Bildung der dichten/porösen Strukturkombination.

20. Dichte/poröse Strukturkombination nach Anspruch 1, die als ein Verabreichungssystem mit verzögerter Freisetzung von Nutzen ist, umfassend: ein vernetztes poröses Element mit einer offenen, verbundenen Porosität, und ein dichtes Element, das mindestens einen Teil des porösen Elements umgibt, wobei zumindest eines der dichten oder porösen Elemente einen physiologisch aktiven Wirkstoff enthält.

21. Verabreichungssystem mit verzögerter Freisetzung nach Anspruch 20, wobei das poröse Element den physiologisch aktiven Wirkstoff enthält.

22. Verabreichungssystem mit verzögerter Freisetzung nach Anspruch 20, wobei das poröse Element eine äußere Oberfläche aufweist, die eine Form mit einem Rohvolumen definiert, wobei das Element in dem gesamten Rohvolumen ein kontinuierliches Netzwerk mit Verstrebungen umfasst, die eine Vielzahl von verbindenden Zwischenräumen definieren, wobei das poröse Element verbindende Zwischenräumen, die sich über das gesamte Volumen erstrecken, und Durchgangsöffnungen durch diese Oberfläche aufweist.

23. Verabreichungssystem mit verzögerter Freisetzung nach Anspruch 20, wobei die mittleren Zwischenräume weiter sind als die der Verstrebungen.

24. Verabreichungssystem mit verzögerter Freisetzung nach Anspruch 20, wobei die porösen und dichten Elemente eine Keramik umfassen.

25. Verabreichungssystem mit verzögerter Freisetzung nach Anspruch 21, wobei das dichte Element einen Teil des porösen Elements umgibt und die Abgabe des physiologisch aktiven Wirkstoffs aus dem porösen Element durch die Dicke des das poröse Element umgebenden, dichten Elements gesteuert wird.

26. Verabreichungssystem mit verzögerter Freisetzung nach Anspruch 21, wobei das dichte Element einen Teil des porösen Elements umgibt und die Abgabe des physiologisch aktiven Wirkstoffs aus dem porösen Element durch die Oberfläche des porösen Elements, das von dem dichten Element bedeckt ist, gesteuert wird.

27. Verabreichungssystem mit verzögerter Freisetzung nach Anspruch 21, wobei das dichte Element ein biologisch resorbierbares Material ist und die Abgabe des physiologisch aktiven Wirkstoffs aus dem porösen Element durch Bioabsorption des dichten Elements gesteuert wird.

28. Verabreichungssystem mit verzögerter Freisetzung nach Anspruch 24, wobei die dichte/poröse Strukturkombination durch ein Verfahren erzeugt wird, umfassend: Bereitstellen eines porösen Elements; Bereitstellen eines dichten Keramik- oder Metallelements, das aus einem Material gebildet ist und eine Sinterdichte von mindestens 95 % aufweist; Bereitstellen einer klebenden Phase, Verbinden mindestens einer Oberfläche des porösen Elements und des dichten Elements mit der klebenden Phase, wodurch die klebende Phase in das poröse Element eindringt und eine Verbindungszone bildet; Trocknen der klebenden Phase zur Bildung der dichten/porösen Strukturkombination; und Sintern des kombinierten dichten und porösen Elements mit der Verbindungszone zur Bildung der dichten/porösen Strukturkombination.

29. Verfahren zur Erzeugung einer dichten/porösen Strukturkombination nach Anspruch 1, umfassend:
(a) Bereitstellen eines porösen Elements mit einer äußeren Oberfläche, die eine Form mit einem Rohvolumen definiert, wobei das Element im gesamten Rohvolumen ein Netzwerk mit Verstrebungen umfasst, die eine Vielzahl von verbindenden Zwischenräumen definieren, wobei dieses Netzwerk eine vernetzte Struktur mit 3-3-Konnektivität aufweist, wobei dieses Element verbindende Zwischenräume aufweist, die sich durch das gesamte Volumen erstrecken und sich durch diese Oberfläche hindurch öffnen; und wobei das Material dieses porösen Elements Keramiken, Metalle und Kombinationen davon umfasst;
(b) Bereitstellen eines dichten, aus einem Material gebildeten Elements, das eine Sinterdichte von mindestens 95 % aufweist;
(c) Bereitstellen einer klebenden Phase;
(d) Verbinden mindestens einer Oberfläche des porösen Elements und des dichten Elements mit der klebenden Phase, wodurch die klebende Phase in das poröse Element eindringt und eine Verbindungszone bildet;
(e) Trocknen der klebenden Phase zur Bildung einer dichten/porösen Strukturkombination; und
(f) Sintern des kombinierten dichten und porösen Elements mit der Verbindungszone zur Bildung der dichten/porösen Strukturkombination.

30. Verfahren zur Erzeugung einer dichten/porösen Strukturkombination nach Anspruch 29, wobei die klebende Phase eine Dispersion eines Keramik- oder Metallpulvers, ein Bindemittel und ein Lösungsmittel in der Form eines Schlickers umfasst.

31. Spritzgussverfahren zur Erzeugung einer dichten/porösen Strukturkombination nach Anspruch 3, umfassend:
(a) Bereitstellen eines porösen Elements in einem Grünzustand, das aus einem organischen, vernetzten Schaum erzeugt wurde;
(b) Einführen des porösen Elements in eine Gussform;
(b) Bereitstellen einer ersten Dispersion, umfassend ein Keramik- oder Metallpulver und ein Bindemittel, und Bilden eines Rohmaterials;
(d) Spritzgießen des Rohmaterials in das Werkzeug für eine Zeit und unter einem Druck, der ausreicht, das Rohmaterial in mindestens einen Teil des porösen Elements zu zwingen, um eine gegossene, dichte/poröse Grünstruktur zu bilden;
(e) Entnehmen der gegossenen dichten/porösen Grünstruktur aus dem Werkzeug;
(f) Entbindern der gegossenen Struktur; und
(e) Sintern der gegossenen, grünen dichten/porösen Strukturkombination zur Bildung der dichten/porösen Strukturkombination.

32. Verfahren nach Anspruch 31, wobei der Werkzeughohlraum mindestens eine Abmessung aufweist, die größer als die entsprechende Abmessung des porösen Elements ist.

33. Verfahren nach Anspruch 31, wobei der Schritt des Spritzgusses des Rohmaterials das poröse Grünelement auf ein Volumen komprimiert, das kleiner als das unkomprimierte Volumen des porösen Elements ist, und der Schritt des Entbinderns der geformten dichten/porösen Grünstruktur das poröse Grünelement expandiert, sodass die gesinterte dichte/poröse Struktur eine Abmessung aufweist, die größer ist als die entsprechende Abmessung des Werkzeughohlraums.

34. Verfahren nach Anspruch 31, des Weiteren umfassend: Füllen zumindest eines Teils der Zwischenräume des porösen Grünelements mit einem Material, das die Form des porösen Grünelements während des Spritzgusses aufrechterhält, und Entfernen des Materials aus dem porösen Grünelement, sodass die Abmessungen des porösen Grünelements im Wesentlichen identisch zu den Abmessungen vor dem Spritzguss verbleiben.

35. Verfahren nach Anspruch 31, wobei das poröse Element in einem Grünzustand Kanäle zum Aufnehmen der Rohmaterialflussfront umfasst, wenn der Werkzeughohlraum gefüllt wird.

36. Verfahren nach Anspruch 34, wobei das Material ein Wachs ist und das Entfernen des Materials aus dem porösen Grünelement die Anwendung eines Lösungsmittels an dem porösen Grünelement umfasst.

37. Verfahren nach Anspruch 34, wobei das Material ein Polymer ist und das Entfernen des Materials aus dem porösen Grünelement die Anwendung eines Lösungsmittels an dem porösen Grünelement umfasst.

38. Verfahren nach Anspruch 34, wobei das Material ein Salz ist und das Entfernen des Materials aus dem porösen Grünelement die Anwendung eines Lösungsmittels an dem porösen Grünelement umfasst.

39. Verfahren nach Anspruch 34, wobei das Material ein Wachs ist und das Entfernen des Materials aus dem porösen Grünelement die Anwendung von Hitze an dem porösen Grünelement umfasst.

40. Verfahren nach Anspruch 34, wobei das Material ein Polymer ist und das Entfernen des Materials aus dem porösen Grünelement die Anwendung von Hitze an dem porösen Grünelement umfasst.

41. Verfahren nach Anspruch 34, wobei das Material ein Salz ist und das Entfernen des Materials aus dem porösen Grünelement die Anwendung von Hitze an dem porösen Grünelement umfasst.

42. Schlickergussverfahren nach Anspruch 29 zur Erzeugung einer dichten/porösen Strukturkombination nach Anspruch 3, umfassend:
(a) Bereitstellen einer porösen Gussform;
(b) Bereitstellen einer ersten Dispersion aus einem Keramik-oder Metallpulver, einem Bindemittel und einem Lösungsmittel zur Bildung eines Schlickers;
(c) Gießen des Schlickers in die Gussform, wobei das Lösungsmittel im Schlicker durch die Gussform durch Kapillarwirkung entfernt wird, um ein dichtes Grünelement zu bilden;
(d) gegebenenfalls Zugeben von zusätzlichem Schlicker, um eine oder mehrere Abmessungen des dichten Elements anzupassen;
(e) Bereitstellen eines porösen Grünelements;
(g) In-Kontakt-Bringen des porösen Grünelements mit dem dichten Grünelement, während mindestens eine Oberfläche des dichten Elements mit dem Schlicker benetzt ist;
(h) Trocknen zur Bildung einer dichten/porösen Grünstruktur;
(i) Entnehmen der dichten/porösen Grünstruktur; und
(j) Sintern der dichten/porösen Struktur zur Bildung der dichten/porösen Strukturkombination.

43. Verfahren nach Anspruch 42, des Weiteren umfassend das Entfernen von überschüssigem Schlicker aus der Gussform, wenn eine gewünschte Abmessung erreicht ist und wobei der nasse Schlicker auf der mindestens einen Oberfläche des dichten Elements aus dem zur Bildung des festen Elements verwendeten Schlicker stammt.

44. Verfahren nach Anspruch 42, des Weiteren umfassend das Zugeben von zusätzlichem Schlicker, um ein Minimum an nassem Schlicker zu halten, um eine Verbindungszone zwischen dem porösen und dem dichten Element bereitzustellen.

45. Verfahren nach Anspruch 29 zur Erzeugung einer dichten/porösen Strukturkombination nach Anspruch 3, umfassend:
(a) Bereitstellen eines porösen Elements in einem Grünzustand;
(b) Bereitstellen einer ersten Dispersion aus einem Keramik-oder Metallpulver, einem Bindemittel und einem Lösungsmittel zur Bildung eines Schlickers;
(c) Beschichten mindestens eines Teils einer Oberfläche des porösen Elements mit dem Schlicker, wodurch der Schlicker zumindest teilweise in mindestens einen Teil des porösen Elements eindringt, um die Verbindungszone zu bilden, und aus dem Schlicker ein der Verbindungszone benachbartes dichtes Element gebildet wird, um eine dichte/poröse Struktur zu bilden;
(d) gegebenenfalls weiteres Zugeben von Auftragungen von Schlicker zu dem porösen Element;
(e) Trocknen der dichten/porösen Strukturkombination zur Bildung einer dichten/porösen Grünstruktur; und
(f) Sintern der dichten/porösen Grünstruktur zur Bildung der dichten/porösen Strukturkombination.

46. Verfahren nach Anspruch 45, wobei die Beschichtung auf mindestens einer Oberfläche das Aufpinseln des Schlickers auf mindestens eine Oberfläche umfasst.

47. Verfahren nach Anspruch 29 zur Erzeugung einer dichten/porösen Strukturkombination nach Anspruch 21, umfassend:
(a) Bereitstellen eines gesinterten porösen Elements mit einer äußeren Oberfläche, die eine Form mit einem Rohvolumen definiert, wobei das Element in dem gesamten Rohvolumen ein Netzwerk mit Verstrebungen umfasst, die eine Vielzahl von verbindenden Zwischenräumen definieren, wobei dieses Netzwerk eine vernetzte Struktur mit 3-3-Konnektivität aufweist, wobei dieses Element verbindende Zwischenräume aufweist, die sich durch das gesamte Volumen erstrecken und sich durch diese Oberfläche hindurch öffnen; und wobei das Material des porösen Elements Keramiken, Metalle und Kombinationen davon umfasst;
(b) Bereitstellen eines gesinterten, dichten, aus einem Material gebildeten Elements, das eine Sinterdichte von mindestens 95 % aufweist;
(c) Bereitstellen einer klebenden Phase;
(d) Verbinden mindestens einer Oberfläche des gesinterten porösen Elements und des gesinterten dichten Elements mit der klebenden Phase, wodurch die klebende Phase in das poröse Element eindringt und eine Verbindungszone bildet; und
(e) gegebenenfalls Härten, Trocknen und/oder Erhitzen der klebenden Phase zur Bildung der dichten/porösen Strukturkombination.

48. Verfahren nach Anspruch 47 zur Erzeugung einer dichten/porösen Strukturkombination, wobei die klebende Phase eine Dispersion eines Keramik- oder Metallpulvers, eines Bindemittels und eines Lösungsmittels in der Form eines Schlickers umfasst.

## Revendications

1. Structure dense/poreuse combinée utilisée comme substitut osseux, comprenant : un élément poreux ayant une surface externe définissant une forme présentant un volume apparent, l'élément comprenant une ossature continue ayant des entretoises définissant une pluralité d'interstices d'interconnexion, au sein du volume apparent, ladite ossature ayant une structure réticulée selon une connectivité de type 3-3, ledit élément poreux ayant des interstices d'interconnexion s'étendant au sein dudit volume et s'ouvrant à travers ladite surface, et le matériau dudit élément poreux comprenant des céramiques, des métaux et leurs combinaisons ; un élément céramique ou métallique dense formé d'un matériau et ayant une masse volumique frittée d'au moins 95 % venant en contact avec au moins une partie de l'élément poreux ; une phase de liaison ; et une zone d'interconnexion joignant au moins une surface de l'élément poreux et l'élément dense, de sorte que la phase de liaison pénètre dans l'élément poreux.

2. Structure dense/poreuse combinée selon la revendication 1, dans laquelle les interstices sont, en moyenne, plus larges que les épaisseurs des entretoises.

3. Structure dense/poreuse combinée selon la revendication 1, comprenant une zone d'interconnexion formée par interpénétration du matériau formant l'élément dense dans l'élément poreux.

4. Structure dense/poreuse combinée selon la revendication 3, dans laquelle le volume apparent présente une surface tournée vers l'intérieur et la partie dense vient en contact avec au moins une partie de la surface tournée vers l'intérieur.

5. Structure dense/poreuse combinée selon la revendication 3, dans laquelle les éléments poreux et dense comprennent une céramique.

6. Structure dense/poreuse combinée selon la revendication 3, dans laquelle l'élément poreux présente une porosité de 80 % ou plus.

7. Structure dense/poreuse combinée selon la revendication 3, dans laquelle les interstices sont, en moyenne, plus larges que les épaisseurs des entretoises.

8. Structure dense/poreuse combinée selon la revendication 3, formée par un procédé comprenant les étapes consistant à :
mettre en oeuvre un élément poreux à l'état cru ; mettre en oeuvre une première dispersion d'une poudre céramique ou métallique, d'un liant et d'un solvant de manière à former une barbotine ; mettre en contact la barbotine avec au moins une partie de l'élément poreux, de sorte que la barbotine pénètre au moins partiellement dans au moins une partie de l'élément poreux pour former une zone d'interconnexion et avec un élément dense adjacent à la zone d'interconnexion ; et effectuer le frittage de la structure dense/poreuse combinée.

9. Structure dense/poreuse combinée selon la revendication 8, dans laquelle les éléments poreux et dense comprennent une céramique.

10. Structure dense/poreuse combinée utile comme structure osseuse artificielle comprenant un élément ayant la forme d'un os naturel ou d'une partie d'un os naturel et une section transversale qui comprend la structure dense/poreuse combinée selon la revendication 3 ayant une partie poreuse interne formée de l'élément poreux de manière à imiter la structure spongieuse de l'os ; et une partie externe dense enveloppant complètement la partie interne poreuse formée à partir de l'élément dense dans le but d'imiter la structure compact de l'os.

11. Structure dense/poreuse combinée utile comme structure osseuse artificielle selon la revendication 10, sous la forme d'un élément de remplacement pour un segment d'os long.

12. Structure dense/poreuse combinée utile comme structure osseuse artificielle selon la revendication 10, sous la forme d'un segment métaphysaire ou diaphysaire d'un os.

13. Structure dense/poreuse combinée utile comme structure osseuse artificielle selon la revendication 10, présentant la forme d'un os entier.

14. Structure dense/poreuse combinée utile comme structure osseuse artificielle selon la revendication 10, présentant la forme d'un corps vertébral.

15. Matériau pour substitut osseux comprenant une structure dense/poreuse combinée selon la revendication 3.

16. Structure dense/poreuse combinée selon la revendication 1, comprenant un élément poreux fritté.

17. Structure dense/poreuse combinée selon la revendication 16, dans laquelle les interstices sont, en moyenne, plus larges que les épaisseurs des entretoises.

18. Structure dense/poreuse combinée selon la revendication 16, dans laquelle la structure dense/poreuse combinée est produite par un procédé comprenant les étapes consistant à : mettre en oeuvre un élément poreux fritté ; mettre en oeuvre un élément céramique ou métallique dense fritté formé d'un matériau et ayant une masse volumique frittée d'au moins 95 % ; mettre en oeuvre une phase de liaison ; joindre au moins une surface de l'élément poreux fritté et l'élément dense fritté à la phase de liaison, de sorte que la phase de liaison pénètre dans l'élément poreux et forme une zone d'interconnexion ; et (e) durcir, sécher et/ou chauffer, si nécessaire, la phase de liaison de manière à former la structure dense/poreuse combinée.

19. Structure dense/poreuse combinée selon la revendication 3, dans laquelle la structure dense/poreuse combinée est produite par un procédé comprenant les étapes consistant à :
(a) mettre en oeuvre un élément poreux dans un état cru ;
(b) mettre en oeuvre une dispersion comprenant une poudre céramique ou métallique et un liant ;
(c) mettre en contact la dispersion avec l'élément poreux, de sorte que la barbotine pénètre au moins partiellement dans au moins une partie de l'élément poreux pour former une zone d'interconnexion et avec un élément dense formé à partir de la dispersion et adjacent à la zone d'interconnexion pour former une structure dense/poreuse combinée ;
(d) solidifier la structure dense/poreuse combinée pour former une structure dense/poreuse combinée crue ; et
(e) fritter la structure dense/poreuse combinée crue pour former la structure dense/poreuse combinée.

20. Structure dense/poreuse combinée selon la revendication 1 utile comme système d'administration à libération prolongée comprenant : un élément poreux réticulé ayant une porosité à pores interconnectés ouverts ; et un élément dense enveloppant au moins une partie de l'élément poreux, dans laquelle au moins l'un ou l'autre des éléments dense et poreux contient un agent actif au plan physiologique.

21. Système d'administration à libération prolongée selon la revendication 20, dans lequel l'élément poreux contient l'agent actif au plan physiologique.

22. Système d'administration à libération prolongée selon la revendication 20, dans lequel l'élément poreux présente une surface externe définissant une forme ayant un volume apparent, l'élément comprenant une ossature continue ayant des entretoises définissant une pluralité d'interstices d'interconnexion au sein du volume apparent, et ledit élément poreux ayant des interstices d'interconnexion s'étendant au sein dudit volume et s'ouvrant à travers ladite surface.

23. Système d'administration à libération prolongée selon la revendication 20, dans lequel les interstices sont, en moyenne, plus larges que les épaisseurs des entretoises.

24. Système d'administration à libération prolongée selon la revendication 20, dans lequel l'élément poreux et l'élément dense comprennent une céramique.

25. Système d'administration à libération prolongée selon la revendication 21, dans lequel l'élément dense enveloppe une partie de l'élément poreux et la libération de l'agent actif au plan physiologique de l'élément poreux est contrôlée par l'épaisseur de l'élément dense enveloppant l'élément poreux.

26. Système d'administration à libération prolongée selon la revendication 21, dans lequel l'élément dense enveloppe une partie de l'élément poreux et la libération de l'agent actif au plan physiologique de l'élément poreux est contrôlée par la surface spécifique de l'élément poreux enveloppé par l'élément dense.

27. Système d'administration à libération prolongée selon la revendication 21, dans lequel l'élément dense est un matériau biorésorbable et l'administration de l'agent actif au plan physiologique de l'élément poreux est contrôlée par la bioabsorption de l'élément dense.

28. Système d'administration à libération prolongée selon la revendication 24, dans lequel la structure dense/poreuse combinée est produite par un procédé comprenant les étapes consistant à : mettre en oeuvre un élément poreux ; mettre en oeuvre un élément céramique ou métallique dense formé d'un matériau et ayant une masse volumique frittée d'au moins 95 % ; mettre en oeuvre une phase de liaison ; joindre au moins une surface de l'élément poreux et l'élément dense à la phase de liaison, de sorte que la phase de liaison pénètre dans l'élément poreux et forme une zone d'interconnexion ; sécher la phase de liaison pour former la structure dense/poreuse combinée ; et fritter l'élément dense et poreux combiné avec la zone d'interconnexion pour former la structure dense/poreuse combinée.

29. Procédé pour produire une structure dense/poreuse combinée selon la revendication 1, comprenant les étapes consistant à :
(a) mettre en oeuvre un élément poreux ayant une surface externe définissant une forme présentant un volume apparent, l'élément comprenant une ossature ayant des entretoises définissant une pluralité d'interstices d'interconnexion au sein du volume apparent, ladite ossature ayant une structure réticulée selon une connectivité de type 3-3, ledit élément ayant des interstices d'interconnexion s'étendant au sein dudit volume et s'ouvrant à travers ladite surface, et le matériau dudit élément poreux comprenant des céramiques, des métaux et des combinaisons de ceux-ci ;
(b) mettre en oeuvre un élément dense formé d'un matériau et ayant une masse volumique frittée d'au moins 95 % ;
(c) mettre en oeuvre une phase de liaison ;
(d) joindre au moins une surface de l'élément poreux et l'élément dense à la phase de liaison, de sorte que la phase de liaison pénètre dans l'élément poreux et forme une zone d'interconnexion ;
(e) sécher la phase de liaison pour former la structure dense/poreuse combinée ; et
(f) fritter l'élément dense et poreux combiné avec la zone d'interconnexion de manière à former la structure dense/poreuse combinée.

30. Procédé pour produire une structure dense/poreuse combinée selon la revendication 29, dans lequel la phase de liaison comprend une dispersion à base d'une poudre céramique ou métallique, d'un liant et d'un solvant, sous la forme d'une barbotine.

31. Procédé de moulage par injection pour produire une structure dense/poreuse combinée selon la revendication 3, comprenant les étapes consistant à :
(a) mettre en oeuvre un élément poreux dans un état cru produit à partir d'une mousse organique réticulée ;
(b) insérer l'élément poreux dans un moule ;
(c) mettre en oeuvre une première dispersion comprenant une poudre céramique ou métallique et un liant et former une charge d'alimentation ;
(d) mouler par injection la charge d'alimentation dans le dispositif de moulage pendant une période de temps et sous une pression suffisantes pour refouler la charge d'alimentation dans au moins une partie de l'élément poreux de manière à former une structure dense/poreuse moulée crue ;
(e) retirer la structure dense/poreuse moulée crue du dispositif de moulage ;
(f) dégager la structure moulée ; et
(g) fritter la structure dense/poreuse combinée moulée crue pour former la structure dense/poreuse combinée.

32. Procédé selon la revendication 31, dans lequel la cavité du dispositif de moulage présente au moins une dimension plus grande qu'une dimension correspondante de l'élément poreux.

33. Procédé selon la revendication 31, dans lequel l'étape consistant à mouler par injection la charge d'alimentation comprime l'élément poreux cru en un volume plus petit que le volume non comprimé de l'élément poreux, et l'étape consistant à dégager la structure dense/poreuse moulée crue dilate l'élément poreux cru, de sorte que la structure dense/poreuse frittée présente une dimension plus grande qu'une dimension correspondante de la cavité du dispositif de moulage.

34. Procédé selon la revendication 31, comprenant en outre les étapes consistant à : remplir au moins une partie des interstices d'un élément poreux cru avec un matériau qui maintient la forme de l'élément poreux cru au cours du moulage par injection ; et retirer le matériau de l'élément poreux cru, de sorte que les dimensions de l'élément poreux cru restent sensiblement identiques aux dimensions observées avant le moulage par injection.

35. Procédé selon la revendication 31, dans lequel l'élément poreux à l'état cru comprend des canaux pour recevoir le front de courant de la charge d'alimentation à mesure que la cavité du dispositif de moulage se remplit.

36. Procédé selon la revendication 34, dans lequel le matériau est une cire et l'élimination du matériau de l'élément poreux cru comprend l'application d'un solvant à l'élément poreux cru.

37. Procédé selon la revendication 34, dans lequel le matériau est un polymère et l'élimination du matériau de l'élément poreux cru comprend l'application d'un solvant à l'élément poreux cru.

38. Procédé selon la revendication 34, dans lequel le matériau est un sel et l'élimination du matériau de l'élément poreux cru comprend l'application d'un solvant à l'élément poreux cru.

39. Procédé selon la revendication 34, dans lequel le matériau est une cire et l'élimination du matériau de l'élément poreux cru comprend l'application de chaleur à l'élément poreux cru.

40. Procédé selon la revendication 34, dans lequel le matériau est un polymère et l'élimination du matériau de l'élément poreux cru comprend l'application de chaleur à l'élément poreux cru.

41. Procédé selon la revendication 34, dans lequel le matériau est un sel et l'élimination du matériau de l'élément poreux cru comprend l'application de chaleur à l'élément poreux cru.

42. Procédé de coulée en barbotine selon la revendication 29, pour la production d'une structure dense/poreuse combinée selon la revendication 3, comprenant les étapes consistant à :
(a) mettre en oeuvre un moule poreux ;
(b) mettre en oeuvre une première dispersion comprenant une poudre céramique ou métallique, un liant et un solvant de manière à former une barbotine ;
(c) verser la barbotine dans le moule, de sorte que le solvant dans la barbotine soit éliminé à travers le moule par effet de capillarité pour former un élément dense cru ;
(d) éventuellement, ajouter une quantité de barbotine supplémentaire pour ajuster une ou plusieurs dimensions de l'élément dense ;
(e) mettre en oeuvre un élément poreux cru ;
(g) mettre en contact l'élément poreux cru avec l'élément dense cru, tandis qu'au moins une surface de l'élément dense est mouillée à la barbotine ;
(h) sécher pour former une structure dense/poreuse crue ;
(i) retirer la structure dense/poreuse crue ; et
(e) fritter la structure dense/poreuse pour former la structure dense/poreuse combinée.

43. Procédé selon la revendication 42, comprenant en outre l'élimination de la barbotine en excès du moule lorsqu'une dimension souhaitée est obtenue et dans lequel la barbotine mouillée sur la au moins une surface de l'élément dense provient de la barbotine utilisée pour former l'élément solide.

44. Procédé selon la revendication 42, comprenant en outre l'addition d'une quantité de barbotine supplémentaire pour retenir un minimum de barbotine mouillée dans le but de mettre en oeuvre une zone d'interconnexion entre les éléments poreux et dense.

45. Procédé selon la revendication 29, pour produire une structure dense/poreuse combinée selon la revendication 3, comprenant les étapes consistant à :
(a) mettre en oeuvre un élément poreux à l'état cru ;
(b) mettre en oeuvre une première dispersion comprenant une poudre céramique ou métallique, un liant et un solvant de manière à former une barbotine ;
(c) revêtir au moins une partie d'une surface de l'élément poreux avec la barbotine, de sorte que la barbotine pénètre au moins partiellement dans au moins une partie de l'élément poreux pour former la zone d'interconnexion et qu'un élément dense soit formé à partir de la barbotine et sous forme adjacente à la zone d'interconnexion pour fournir une structure dense/poreuse ;
(d) éventuellement, ajouter des revêtements supplémentaires de barbotine à l'élément poreux ;
(e) sécher la structure dense/poreuse combinée de manière à former une structure dense/poreuse combinée crue ; et
(f) fritter la structure dense/poreuse crue pour former la structure dense/poreuse combinée.

46. Procédé selon la revendication 45, dans lequel le revêtement d'au moins une surface comprend le revêtement à la brosse de la barbotine sur la au moins une surface.

47. Procédé selon la revendication 29, pour la production d'une structure dense/poreuse combinée selon la revendication 21, comprenant les étapes consistant à :
(a) mettre en oeuvre un élément poreux fritté ayant une surface externe définissant une forme présentant un volume apparent, l'élément comprenant une ossature ayant des entretoises définissant une pluralité d'interstices d'interconnexion au sein du volume apparent, ladite ossature ayant une structure réticulée selon une connectivité de type 3-3, ledit élément ayant des interstices d'interconnexion s'étendant au sein dudit volume et s'ouvrant à travers ladite surface, et le matériau dudit élément poreux comprenant des céramiques, des métaux et des combinaisons de ceux-ci ;
(b) mettre en oeuvre un élément dense fritté formé d'un matériau et ayant une masse volumique frittée d'au moins 95 % ;
(c) mettre en oeuvre une phase de liaison ;
(d) joindre au moins une surface de l'élément poreux fritté et l'élément dense fritté à la phase de liaison, de sorte que la phase de liaison pénètre dans l'élément poreux et forme une zone d'interpénétration ; et
(e) durcir, sécher et/ou chauffer, si nécessaire, la phase de liaison de manière à former la structure dense/poreuse combinée.

48. Procédé selon la revendication 47, pour produire une structure dense/poreuse combinée, dans lequel la phase de liaison comprend une dispersion comprenant une poudre céramique ou métallique, un liant et un solvant sous la forme d'une barbotine.
